(19) Europäisches Patentamt
European Patent Office
Office européen des brevets

(11) EP 1 412 361 B1

(12) EUROPÄISCHE PATENTSCHRIFT

(45) Veröffentlichungstag und Bekanntmachung des Hinweises auf die Patenterteilung:
**23.07.2008 Patentblatt 2008/30**

(21) Anmeldenummer: **02764838.5**

(22) Anmeldetag: **05.08.2002**

(51) Int Cl.:
***C07D 491/04*** *(2006.01)* ***A61K 31/445*** *(2006.01)*
***A61K 31/34*** *(2006.01)* ***A61P 25/04*** *(2006.01)*
***A61P 25/06*** *(2006.01)* ***A61P 25/00*** *(2006.01)*

(86) Internationale Anmeldenummer:
**PCT/EP2002/008886**

(87) Internationale Veröffentlichungsnummer:
**WO 2003/014124 (20.02.2003 Gazette 2003/08)**

(54) **SUBSTITUIERTE 5,6,6A,11B-TETRAHYDRO-7-OXA-5-AZA-BENZO[C]FLUOREN-6-CARBONSÄUREDERIVATE ALS NMDA-ANTAGONISTEN**

SUBSTITUTED 5,6,6A,11B-TETRAHYDRO-7-OXA-5-AZA-BENZO[C]FLUORENE-6-CARBOXYLIC ACID DERIVATIVES SERVING AS NMDA ANTAGONISTS

DERIVES D'ACIDE 5,6,6A,11B-TETRAHYDRO-7-OXA-5-AZA-BENZO[C]FLUORENE-6-CARBOXYLIQUE SUBSTITUES COMME ANTAGONISTES NMDA

(84) Benannte Vertragsstaaten:
**AT BE BG CH CY CZ DE DK EE ES FI FR GB GR IE IT LI LU MC NL PT SE SK TR**
Benannte Erstreckungsstaaten:
**LT LV RO SI**

(30) Priorität: **03.08.2001 DE 10137487**

(43) Veröffentlichungstag der Anmeldung:
**28.04.2004 Patentblatt 2004/18**

(73) Patentinhaber: **Grünenthal GmbH**
**52078 Aachen (DE)**

(72) Erfinder:
- **GERLACH, Matthias**
  **63636 Brachttal (DE)**
- **PRZEWOSNY, Michael**
  **52062 Aachen (DE)**
- **ENGLBERGER, Werner, Günter**
  **52223 Stolberg (DE)**
- **REISSMÜLLER, Elke**
  **33617 Bielefeld (DE)**
- **BLOMS-FUNKE, Petra**
  **52146 Würselen (DE)**
- **MAUL, Corinna**
  **52066 Aachen (DE)**
- **JAGUSCH, Utz-Peter**
  **52066 Aachen (DE)**

(56) Entgegenhaltungen:
**EP-A- 0 386 839** **WO-A-01/58875**
**WO-A-98/34111** **WO-A-98/42673**
**WO-A-99/64411**

- **R. W. CARLING ET AL.: "2-Carboxytetrahydroquinolines. Conformational and Stereochemical Requirements for Antagonism of the Glycine Site on the NMDA Receptor" J. MED. CHEM., Bd. 35, Nr. 11, 1992, Seiten 1942-1953, XP001002472**
- **E. BORRIONE ET AL.: "Synthesis and Cycloaddition Reactions of Ethyl Glyoxylate Imines. Synthesis of Substituted Furo[3,2-c] quinolines and 7H-Indeno[2,1-c]quinolines" J. HETEROCYCL. CHEM., Bd. 25, 1988, Seiten 1831-1835, XP000670083**

EP 1 412 361 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft substituierte 5,6,6a,11b-Tetrahydro-7-oxa-5-aza-benzo[c]fluoren-6-carbonsäurederivate, sowie Verfahren zu deren Herstellung, Arzneimittel enthaltend diese Verbindungen und deren Verwendung zur Herstellung von Arzneimitteln für bestimmte Indikationen, insbesondere zur Behandlung von Schmerz.

Die Behandlung chronischer und nicht chronischer Schmerzzustände hat in der Medizin eine große Bedeutung. Es besteht ein weltweiter Bedarf an gut wirksamen Schmerztherapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist. Dies zeigt sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Proteingekoppelten Rezeptoren gehören. Die biochemische und pharmakologische Charakterisierung von Subtypen dieser Rezeptoren hat nun die Hoffnung geweckt, daß subtypenspezifische Opioide über ein anderes Wirkungs-/Nebenwirkungsprofil als z.B. Morphin verfügen. Weitere pharmakologische Untersuchungen haben inzwischen die Existenz mehrerer Subtypen dieser Opioidrezeptoren ($\mu_1$, $\mu_2$, $\kappa_1$, $\kappa_2$, $\kappa_3$, $\delta_1$ und $\delta_2$) wahrscheinlich gemacht. Daneben gibt es weitere Rezeptoren und Ionenkanäle, die wesentlich an dem System der Schmerzentstehung und Schmerzweiterleitung beteiligt sind. Besonders wichtig ist dabei der NMDA-Ionenkanal: Über ihn läuft ein wesentlicher Teil der Kommunikation von Synapsen ab. Durch diesen Kanal wird der Calcium-Ionenaustausch zwischen neuronaler Zelle und seiner Umgebung gesteuert.

Kenntnisse über die physiologische Bedeutung von Ionenkanal-selektiven Substanzen sind durch die Entwicklung der patch-clamp-Technik gewonnen worden. So läßt sich eindeutig die Wirkung von NMDA-Antagonisten auf den Einfluß von Calcium-Ionen in das Zellinnere nachweisen. Es stellte sich auch dabei heraus, daß diese Substanzen über ein eigenständiges antinociceptives Potential verfügen (z.B. Ketamin). Wichtig dabei ist, daß der Wirkmechanismus ein ganz anderer ist, wie beispielsweise bei den Opiaten, denn durch NMDA-Antagonisten wird direkt in den entscheidenden Calciumhaushalt der Zellen bei der Schmerzweiterleitung eingegriffen. Daher besteht erstmalig die Möglichkeit, die Behandlung von neuropathischen Schmerzformen erfolgreich durchzuführen.

Verschiedene NMDA-Antagonisten, wobei es sich in diesem Falle um Tetrahydrochinolinderivate handelte, wurden bereits in den Artikeln J. Med. Chem. (1992) 35, 1954-1968, J. Med. Chem. (1992) 35, 1942-1953 und Med. Chem. Res. (1991) 1; 64-73 sowie den Patentanmeldungen EP 386 839, WO 97/12879 A1, WO 98/07704 A1 und WO 98/42673 A1 beschrieben. Dabei wurde insbesondere in den Patentanmeldungen eine Vielzahl von möglichen Indikationen angegeben, unter anderen auch die Schmerztherapie. Die Wirksamkeit und Verwendbarkeit dieser Substanzen ist allerdings weiter offen, so daß hier ein Bedarf nach weiteren Substanzen besteht.

Eine der Erfindung zugrundeliegende Aufgabe bestand darin, analgetisch wirksame Substanzen, insbesondere NMDA-Antagonisten, zur Verfügung zu stellen, die sich zur Schmerztherapie - insbesondere auch chronischer und neuropathischer Schmerzen - eignen. Darüber hinaus sollten diese Substanzen möglichst wenig Nebenwirkungen wie z.B. Übelkeit, Erbrechen, Abhängigkeit, Atemdepression oder Obstipation aufweisen. Entsprechend sind Gegenstand der Erfindung 5,6,6a,11b-Tetrahydro-7-oxa-5-aza-benzo[c]fluoren-6-carbonsäurederivate der allgemeinen Formel I

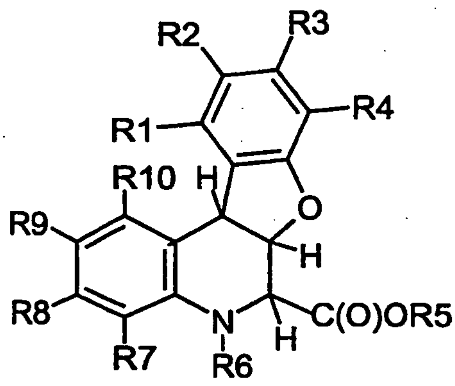

I

in dargestellter Form oder in Form ihrer Säuren oder ihrer Basen oder in Form ihrer Salze, insbesondere der physiologisch verträglichen Salze, oder in Form ihrer Solvate, insbesondere der Hydrate; insbesondere in Form ihrer physiologisch verträglichen Salze mit Kationen oder Basen oder mit Anionen oder Säuren; gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; worin

**$R^1$**, **$R^2$**, **$R^3$** und **$R^4$** unabhängig voneinander ausgewählt sind aus H, F, Cl, Br, I, CN, $NH_2$, $CH_3$, $C_2H_5$, n-Propyl, i-Propyl, i-Butyl, sek-Butyl, n-Butyl, t-Butyl, $CF_3$, $CHF_2$, SH, OH, $OCH_3$, $OC_2H_5$, C(O)OH C(O)$OCH_3$ oder C(O)$OC_2H_5$

und

$R^5$= H,
$R^6$= H,
$R^7$= H,
$R^8$= Cl,
$R^9$= H und
$R^{10}$= Cl ist.

Die erfindungsgemäßen 1,2,3,4-Tetrahydrochinolin-2-carbonsäurederivate zeigen eine deutliche analgetische Wirkung und sind auch NMDA-Antagonisten, die selektiv an der Glycin-Bindungsstelle angreifen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali-und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologischinsbesondere bei Anwendung im Menschen und/ oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydrol1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, a-Liponsäure, Acetylglycin, Acetylsalicylsäure, Hippursäure und/oder Asparaginsäure. Besonders bevorzugt ist das Hydrochlorid-Salz.

Bevorzugte Gegenstände sind insbesondere folgende substituierte 5,6,6a,11b-Tetrahydro-7-oxa-5-aza-benzo [c]fluoren-6-carbonsäurederivate bzw. ihre Salze:

- 1,3-Dichlor-5,6,6a,11b-tetrahydro-7-oxa-5-aza-benzo[c]fluoren-6-carbonsäure,
- 1,3-Dichlor-10-methoxy-5,6,6a,11b-tetrahydro-7-oxa-5-aza-benzo[c]fluoren-6-carbonsäure,
- 1,3-Dichlor-8-methyl-5,6,6a,11b-tetrahydro-7-oxa-5-aza-benzo[c]fluoren-6-carbonsäure,
- 1,3-Dichlor-8-ethyl-5,6,6a,11b-tetrahydro-7-oxa-5-aza-benzo[c]fluoren-6-carbonsäure,
- 1,3-Dichior-8-fluor-5,6,6a,11b-tetrahydro-7-oxa-5-aza-benzo[c]fluoren-6-carbonsäure,
- 1,3,8-Trichlor-5,6,6a,11b-tetrahydro-7-oxa-5-aza-benzo[c]fluoren-6-carbonsäure,
- 8-Brom-1,3-dichlor 5,6,6a,11b-tetrahydro-7-oxa-5-aza- , benzo[c]fluoren-6-carbonsäure,
- 8-Iod-1,3-dichlor-5,6,6a,11b-tetrahydro-7-oxa-5-aza-benzo[c]fluoren-6-carbonsäure,
- 1,3-Dichlor-5,6,6a,11b-tetrahydro-7-oxa-5-aza-benzo[c]fluoren-6,8-dicarbonsäure,
- 1,3-Dichlor-10-methyl-5,6,6a,11b-tetrahydro-7-oxa-5-aza-benzo[c]fluoren-6-carbonsäure,
- 1,3-Dichlor-10-fluor-5,6,6a,11b-tetrahydro-7-oxa-5-aza-benzo[c]fluoren-6-carbonsäure,
- 1,3,10-Trichlor-5,6,6a,11b-tetrahydro-7-oxa-5-aza-benzo[c]fluoren-6-carbonsäure,
- 10-Brom-1,3-dichlor-5,6,6a,11b-tetrahydro-7-oxa-5-aza-benzo[c]fluoren-6-carbonsäure,
- 10-Iod-1,3-dichlor-5,6,6a,11b-tetrahydro-7-oxa-5-aza-benzo[c]fluoren-6-carbonsäure,
- 1,3-Dichlor-5,6,6a,1b-tetrahydro-7-oxa-5-aza-benzo[c]fluoren-6,10-dicarbonsäure oder
- 1,3-Dichlor-10-cyano-5,6,6a,11b-tetrahydro-7-oxa-5-aza-benzo[c]fluoren-6-carbonsäure,

Besonders bevorzugt sind die freien Carbonsäuren oder die Salze der erfindungsgemäßen substituierten 1,2,3,4-Tetrahydrochinolin-2-carbonsäurederivate gemäß Formel I in Form ihrer Alkali-Salze, vorzugsweise der Kalium- oder Natrium-Salze, oder in Form der Salze anorganischer Säuren, vorzugsweise des Hydrochlorids.

Ein weiterer Gegenstand der Erfindung sind auch Verfahren zur Herstellung erfindungsgemäßer Salze eines substituierten 5,6,6a,-11b-Tetrahydro-7-oxa-5-aza-benzo[c]fluoren-6-carbonsäurederivats.

In der Literatur sind verschiedene Verfahren zur Darstellung von Tetrahydrochinolinen, dem Grundkörper der erfindungsgemäßen Verbindungen, beschrieben:

- ein Festphasen-Ansatz (WO 98/34111),
- mehrstufige Prozessführungen (WO 98/42673; Bioorganic and Medicinal Chemistry Lettetters Vol. 2, S. 371, 1992; Journal of Heterocyclic Chemistry Vol. 25, S. 1831, 1988; Journal of the Chemical Society, Perkin Transactions I (1989), Seite 2245) oder
- ein Lewis-Säure-katalysiertes "Eintopf"-Verfahren (Journal of the Chemical Society, Chemical Communications, 1999, S. 651; Journal of the American Chemical Society, Vol. 118, S. 8977, 1996).

Alle diese Verfahren weisen aber klar einige Nachteile auf.

Abweichend von diesen ist das hier beschriebene sog. Grundverfahren ein Trifluoressigsäure vermitteltes - vorzugsweise "Eintopf"- Verfahren, bei dem je eine aromatische Amin-, Aldehyd- und elektronenreiche Olefinkomponente miteinander reagieren.

In dem Grundverfahren werden substituierte 5,6,6a,11b-Tetrahydro-7-oxa-5-aza-benzo[c]fluoren-6-carbonsäurederivate gemäß Formel I hergestellt. Dabei werden Aniline gemäß Formel II,

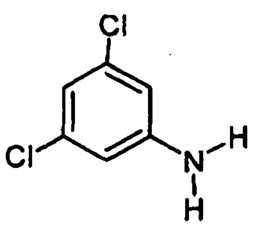
+
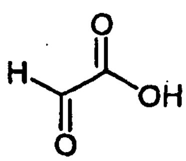
+
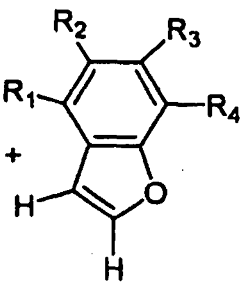

II III IV

mit Glyoxylsäure gemäß Formel **III** und einem Benzofuran gemäß **IV,** in denen $R^1$, $R^2$, $R^3$ und $R^4$ unabhängig voneinander jeweils eine der bereits für Formel I angegebenen Bedeutungen haben oder mit einer Schutzgruppe versehen sind, mit Trifluoressigsäure zwischen 0°C und 100°C umgesetzt werden. Dabei ist es bevorzugt, daß die Reaktionsdauer 0.25 - 12 h, vorzugsweise maximal 2h, beträgt, die Reaktion bevorzugt bei Temperatur zwischen 20 und 40°C, vorzugsweise Raumtemperatur, erfolgt und/oder die Reaktion eine Eintopfreaktion ist.

Ein entscheidender Vorteil des erfindungsgemäßen Verfahrens ist, daß das Verfahren gemäß einer Dominoreaktion (Iminbildung und nachgeschaltete Aza-Diels-Alder-Reaktion) sehr selektiv bei zudem guten Ausbeuten zu den gewünschten Systemen führt.

Ohne einen Knüpfungs- bzw. Abspaltungsschritt durchführen zu müssen, wie im Falle des solid phase-Ansatzes, ferner ohne Aufreinigung der Zwischenstufen - wie im Falle der beschriebenen Lösungschemie - unterscheidet sich das erfindungsgemäße Verfahren neben seiner einfachen Durchführbarkeit ferner durch seine Aufreinigungmethode. Durch mehrmaliges Waschen mit unpolaren Lösungsmitteln, wie beispielsweise n-Hexan lassen sich größtenteils die Produkte in hoher Reinheit erhalten. Anderenfalls gelingt ihre Aufreinigung mittels Säulenchromatographie. Insbesondere lassen sich Verbindungen der Formel I durch die Waschprozesse mit unpolaren Lösungsmitteln - wie beispielsweise *n*Hexan - oder durch Kristallisation ihrer Salze diastereomerenrein erhalten.

Allgemein wird bei einer günstigen Form des Herstellungsverfahrens nach Abschluß der Bildung einer Verbindung nach Formel I die Verbindung mit einer Base oder Säure, die bereits das gewünschte Kation bzw. Anion enthalten kann, in Verbindung gebracht und das entstehende Salz anschließend gereinigt.

Die meisten der hier eingesetzten Reagenzien, insbesondere nach Formel II, III und IV sind käuflich zu erwerben oder können durch einfache, dem Fachmann bekannte Syntheseschritte hergestellt werden.

Unter vielen der genannten Reaktionsbedingungen können OH- SH und $NH_2$-Gruppen möglicherweise unerwünschte Nebenreaktionen eingehen. Es ist daher bevorzugt, diese mit Schutzgruppen zu versehen oder im Falle von $NH_2$ durch $NO_2$ zu ersetzen und vor der Aufreinigung des Endprodukts die Schutzgruppe abzuspalten, bzw. die $NO_2$-Gruppe zu reduzieren. Ein weiterer Gegenstand der Anmeldung ist daher eine Abwandlung der oben beschriebenen Verfahrens, bei dem in den Ausgangsverbindungen mindestens eine OH-Gruppe durch eine $OSi(Ph)_2$tert-Butyl-Gruppe, mindestens eine SH-Gruppe durch eine S-p-Methoxybenzylgruppe und/oder mindestens eine $NH_2$-Gruppe durch eine $NO_2$-Gruppe ersetzt wurde und vor der Aufreinigung des Endprodukts mindestens eine - vorzugsweise alle - $OSi(Ph)_2$tert-Butyl-Gruppe/n, mit Tetrabutylammoniumflluorid in Tetrahydrofuran und/oder mindestens eine - vorzugsweise alle - p-Methoxybenzylgruppe/n mit einem Metallamin, bevorzugt Natriumamin, abgespalten und/oder mindestens eine - vorzugsweise alle - $NO_2$-Gruppe/n zu $NH_2$ reduziert wird.

Weiter sind Carbonsäure- oder Thiocarbonsäure-Gruppen unter den genannten Reaktionsbedingungen unter Umständen nicht stabil, so daß es bevorzugt ist, deren Methylester in den Reaktionen einzusetzen und das Verfahrensprodukt anschließend mit KOH-Lösung bzw. NaOH-Lösung in Methanol bei 40°C - 60°C zu verseifen. Ein weiterer Gegenstand der Erfindung ist daher eine Abwandlung der oben beschriebenen Verfahren, in dem vor der Aufreinigung des Endprodukts ein Verfahrensprodukt mit mindestens einer $C(O)OCH_3$- $OC(O)OCH_3$- und/oder $C(S)OCH_3$-Gruppe mit KOH-Lösung bzw. NaOH-Lösung in Methanol oder Ethanol bei 0°C - 100°C, vorzugsweise 40°C - 60°C, verseift wird.

Daher kann es auch günstig sein, zur Herstellung substituierter 5,6,6a,11b-Tetrahydro-7-oxa-5-aza-benzo[c]fluoren-6-carbonsäurederivate gemäß Formel I mit $R^5$ = H für das Grundverfahren Ausgangsprodukte gemäß Formel III zu verwenden, in denen $R^5 \neq$ H und $R^5$ vorzugsweise Alkyl, insbesondere $CH_3$ und $C_2H_5$ sind, einzusetzen. Nach dem Grundverfahren und auch den möglicherweise daran anschließenden Folgereaktionen wird das Reaktionsprodukt mit einer entsprechenden Base, vorzugsweise NaOH oder KOH, in Ethanol oder Methanol, bei Temperaturen zwischen O-100 °C, vorzugsweise 40°C - 60°C, verseift (Organikum, 1990, S. 418).

Zur Herstellung der Salze insbesondere der physiologisch verträglichen Salze mit Kationen bzw. Basen wird wie folgt vorgegangen:

Ein Äquivalent einer Verbindung gemäß Formel I, vorzugsweise eine Iminosäure, bzw. eine Carbonsäure, insbesondere mit $R^3$ = H, wird in wenig Wasser suspendiert und ein Äquivalent 1-normaler wäßriger Lauge, beispielsweise NaOH oder KOH, zugegeben. Bei schlechter Löslichkeit wird soviel Methanol zugetropft, bis vollständige Lösung eintritt. Nach Rühren bei Raumtemperatur wird im Rotationsverdampfer eingeengt, die verbliebene Lösung bei tiefen Temperaturen in einem Gemisch aus Isopropanol/Trockeneis eingefroren und gefriergetrocknet. Die Salze, insbesondere der Iminosäuren, bzw. Carbonsäuren, vorzugsweise die Natrium- oder Kaliumsalze, werden als meist farblose Feststoffe erhalten. Alternativ ist es auch möglich, die Kalium- bzw. Natriumsalze mit Kalium-bzw. Natriumtrimethylsilanolat herzustellen (E.D. Laganis, B.L. Chenard; Tetrahedron Letters 25, 5831 - 5834 (1984)). Kalium- bzw. Natriumtrimethylsilanolat wird dabei unter Stickstoff in einem organischen Lösungsmittel (z.B. Dichlormethan, Toluol, THF) gelöst und der Ester, bzw. die Säure, in einer Portion zugegeben. Das Reaktionsgemisch wird mehrere Stunden bei Raumtemperatur gerührt und abfiltriert. Der meist farblose Feststoff wird gewaschen und im Vakuum getrocknet. Die Kalium- bzw. Natriumsalze werden als Feststoffe erhalten.

Die erfindungsgemäßen substituierten 5,6,6a,11b-Tetrahydro-7-oxa-5-aza-benzo[c]fluoren-6-carbonsäurederivate sind toxikologisch unbedenklich, so daß sie sich als pharmazeutischer Wirkstoff in Arzneimitteln eignen.

Ein weiterer Gegenstand der Erfindung ist daher auch ein Arzneimittel enthaltend als Wirkstoff mindestens ein erfindungsgemäßes substituiertes 5,6,6a,11b-Tetrahydro-7-oxa-5-aza-benzo[c]fluoren-6-carbonsäurederivat gemäß Formel I in dargestellter Form oder in Form der Säure oder Base oder in Form seiner Salze, insbesondere der physiologisch verträglichen Salze, oder in Form seiner Solvate, insbesondere der Hydrate; insbesondere in Form seiner physiologisch verträglichen Salze mit Kationen oder Basen oder mit Anionen oder Säuren; gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; sowie gegebenenfalls enthaltend geeignete Zusatz-und/oder Hilfsstoffe und/oder gegebenenfalls weitere Wirkstoffe.

Die erfindungsgemäßen Arzneimittel können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden und enthalten neben mindestens einem erfindungsgemäßen substituierten 5,6,6a,11b-Tetrahydro-7-oxa-5-aza-benzo[c]fluoren-6-carbonsäurederivat je nach galenischer Form gegebenenfalls Trägermaterialien Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, peroral, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte 5,6,6a,11b-Tetrahydro-7-oxa-5-aza-benzo[c]fluoren-6-carbonsäurederivate in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten 5,6,6a,11b-Tetrahydro-7-oxa-5-aza-benzo[c]fluoren-6-carbonsäurederivate verzögert freisetzen. Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 2 bis 500 mg/kg wenigstens eines erfindungsgemäßen substituierten 5,6,6a,11b-Tetrahydro-7-oxa-5-aza-benzo[c]fluoren-6-carbonsäurederivats der Formel I appliziert.

Vorzugsweise werden die erfindungsgemäßen substituierten 5,6,6a,11b-Tetrahydro-7-oxa-5-aza-benzo[c]fluoren-6-carbonsäurederivate zur Schmerzbehandlung, insbesondere chronischer und neuropathischer Schmerzen, aber auch bei Migräne eingesetzt, so daß ein weiterer Erfindungsgegenstand die Verwendung mindestens eines erfindungsgemäßen substituierten 5,6,6a,11b-Tetrahydro-7-oxa-5-aza-benzo[c]fluoren-6-carbonsäurederivats gemäß Formel I in dargestellter Form oder in Form der Säure oder Base oder in Form seiner Salze, insbesondere der physiologisch verträglichen Salze, oder in Form seiner Solvate, insbesondere der Hydrate; insbesondere in Form seiner physiologisch verträglichen Salze mit Kationen oder Basen oder mit Anionen oder Säuren; gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; zur Herstellung eines Arzneimittels zur Behandlung von Schmerz, insbesondere des neuropathischen und/oder chronischen Schmerzes, und/oder zur Behandlung von Migräne ist.

Aus der Affinität an den NMDA-Rezeptor ergeben sich weitere Anwendungsgebiete, da NMDA-Antagonisten bekanntermaßen u.a. eine neuroprotektive Wirkung haben und daher auch gut bei mit Neurodegeneration und -schädigung einhergehenden Krankheitsbildern, wie Morbus Parkinson und Morbus Huntington etc. eingesetzt werden können. Weitere Indikationen der erfindungsgemäßen NMDA-Antagonisten sind Epilepsie, Glaukom, Osteoporose, Ototoxizität, die mit Alkohol- und/oder Drogenmißbrauch einhergehenden Entzugserscheinungen, der Schlaganfall, sowie damit zusammenhängend cerebrale Ischämien, cerebrale Infarkten, Hirnödem, Hypoxie, Anoxie, sowie auch der Einsatz zur Anxiolyse und in der Anästhesie. Ein weiterer Gegenstand der Erfindung ist daher die Verwendung mindestens eines erfindungsgemäßen substituierten 5,6,6a, 11b-Tetrahydro-7-oxa-5-aza-benzo[c]fluoren-6-carbonsäurederivats gemäß Formel I; in dargestellter Form oder in Form der Säure oder Base oder in Form seiner Salze, insbesondere der physiologisch verträglichen Salze, oder in Form seiner Solvate, insbesondere der Hydrate; insbesondere in Form seiner physiologisch verträglichen Salze mit Kationen oder Basen oder mit Anionen oder Säuren; gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; zur Herstellung eines Arzneimittels zur Behandlung/Prophylaxe von/bei Epilepsie, Morbus Parkinson, Morbus Huntington, Glaukom, Ototoxizität, Entzugserscheinungen bei Alkohol-und/oder Drogenmißbrauch, Schlaganfall, cerebralen Ischämien, cerebralen Infarkten, Hirnödem, Hypoxie, Anoxie und/oder zur Anxiolyse und/oder Anästhesie.

Überraschenderweise hat es sich herausgestellt, daß die erfindungsgemäßen substituierten 1,2,3,4-Tetrahydrochinolin-2-carbonsäurederivats auch für weitere Indikationen, insbesondere zur Behandlung von Harninkontinenz, Juckreiz, Tinnitus aurium und/oder Diarrhoe sehr geeignet sind. Ein weiterer Gegenstand der Anmeldung ist daher die Verwendung mindestens eines erfindungsgemäßen substituierten 5,6,6a,11b-Tetrahydro-7-oxa-5-aza-benzo[c]fluoren-6-carbonsäurederivats gemäß Formel I; in dargestellter Form oder in Form der Säure oder Base oder in Form seiner Salze, insbesondere der physiologisch verträglichen Salze, oder in Form seiner Solvate, insbesondere der Hydrate; insbesondere in Form seiner physiologisch verträglichen Salze mit Kationen oder Basen oder mit Anionen oder Säuren; gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; zur Herstellung eines Arzneimittels zur Behandlung von Harninkontinenz, Juckreiz, Tinnitus aurium und/oder Diarrhoe

Aber auch in andereren Indikationen sind die erfindungsgemäßen Verbindungen wirksam. Ein weiterer Gegenstand der Anmeldung ist daher die Verwendung mindestens eines erfindungsgemäßen substituierten 5,6,6a,11b-Tetrahydro-7-oxa-5-aza-benzo[c]fluoren-6-carbonsäurederivats gemäß Formel I; in dargestellter Form oder in Form der Säure oder Base oder in Form seiner Salze, insbesondere der physiologisch verträglichen Salze, oder in Form seiner Solvate, insbesondere der Hydrate; insbesondere in Form seiner physiologisch verträglichen Salze mit Kationen oder Basen oder mit Anionen oder Säuren; gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; zur Herstellung eines Arzneimittels zur Behandlung/Prophylaxe von/bei Schizophrenie, Morbus Alzheimer, Psychosen bedingt durch erhöhten Aminosäurespiegel, AIDS-Demens, Encephalomyelitis, Tourette-Syndrom, perinataler Asphyxie inflammatorischen und allergischen Reaktionen, Depressionen, Drogen-und/oder Alkoholmißbrauch, Gastritis, Diabetes, cardiovaskulären Erkrankungen, Atemwegserkrankungen, Husten und/oder seelischen Erkrankungen.

Ein weiterer Gegenstand der Erfindung ist ein Verfahren zur Behandlung eines nichthumanen Säugetieres oder Menschen, das oder der eine Behandlung medizinisch relevanter Symptome benötigt, durch Verabreichung einer therapeutisch wiksamen Dosis eines erfindungsgemäßen substituierten 5,6,6a,11b-Tetrahydro-7-oxa-5-aza-benzo[c]fluoren-6-carbonsäurederivats gemäß Formel I; in dargestellter Form oder in Form der Säure oder Base oder in Form seiner Salze, insbesondere der physiologisch verträglichen Salze, oder in Form seiner Solvate, insbesondere der Hydrate; insbesondere in Form seiner physiologisch verträglichen Salze mit Kationen oder Basen oder mit Anionen oder Säuren; gegebenenfalls in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; oder eines erfindungsgemäßen Arzneimittels. Die Erfindung betrifft insbesondere entsprechende Verfahren zur Behandlung von Schmerz, insbesondere von neuropathischem und/oder chronischem Schmerz und/oder zur Behandlung von Migräne zur Behandlung von Harninkontinenz, Juckreiz, Tinnitus aurium und/oder Diarrhoe, zur Behandlung/Prophylaxe von/bei Epilepsie, Morbus Parkinson, Morbus Huntington, Glaukom, Osteoporose, Ototoxizität, Entzugserscheinungen bei Alkohol- und/oder Drogenmißbrauch, Schlaganfall, cerebralen Ischämien, cerebralen Infarkten, Hirnödem, Hypoxie, Anoxie und/oder zur Anxiolyse und/oder Anästhesie oder zur Behandlung/Prophylaxe von/bei Schizophrenie, Morbus Alzheimer, Psychosen bedingt durch erhöhten Aminosäurespiegel, AIDS-Demens, Encephalomyelitis, Tourette-Syndrom, perinataler Asphyxie inflammatorischen und allergischen Reaktionen, Depressionen, Drogen-und/oder Alkoholmißbrauch, Gastritis, Diabetes, cardiovaskulären Erkrankungen, Atemwegserkrankungen, Husten und/oder seelischen Erkrankungen.
Im folgenden wird die Erfindung weiter durch Beispiele erläutert, ohne sie darauf zu beschränken.

## Beispiele

Die folgenden Beispiele zeigen erfindungsgemäße Verbindungen sowie deren Darstellung und mit diesen durchgeführte Wirksamkeitsuntersuchungen.

Dabei gelten generell folgende Angaben:
Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc.) oder werden nach allgemeinen, dem Fachmann bekannten Herstellungsverfahren synthetisiert. Insbesondere werden einige der Benzofurane, aber auch andere eingesetzte Verbindungen vor der unten beschriebenen Grundsynthese als Synthesebausteine nach bekannten Synthesevorschriften synthetisiert.
Die dünschichtchromatographischen Untersuchungen wurden mit HPTLC-Fertigplatten, Kieselgel 60 F 254, der Firma E. Merck, Darmstadt, durchgeführt.
Die Ausbeuten der hergestellten Verbindungen sind nicht optimiert.

Die Analytik erfolgte über ESI-Massenspektroskopie.

Die Verbindungen sind numeriert, wobei die Angabe in Klammem grundsätzlich der Nummer der zugeordneten Verbindung entspricht.

**Beispiel 0**

**Grundverfahren**

**a)** Je ein Äquivalent Anilinderivat und Trifluoressigsäure werden unter Rühren bei Raumtemperatur in 6 ml/mmol Acetonitril gelöst und anschließend 1,1 Äquivalente Ethylglyoxalat (50 % in Toluol) bzw. 1,1 Äquivalente Glyoxalsäuremonohydrat zugegeben. Nach zehn Minuten werden hierzu 3 Äquivalente der Benzofuran-Komponente zugesetzt und der Verlauf der Reaktion durch Dünnschichtchromatographie verfolgt (Laufmittelsystem Diethylether / Hexan, 1:1). Die Reaktion ist nach 2 Stunden beendet (DC-Kontrolle). Der Reaktionsansatz wird mit einem Überschuß an gesättigter, wäßriger Natriumhydrogencarbonat-Lösung versetzt und die organische Phase drei Mal mit Diethylether extrahiert. Die organische Phase wird mit Wasser neutral gewaschen, über Magnesiumsulfat getrocknet, abfiltriert, mit Diethylether gewaschen und nach Einengen durch Umkristallisation bzw. Kieselgel-Chromatographie isoliert. Die Charakterisierung der 5,6,6a,11b-Tetrahydro-7-oxa-5-aza-benzo[c]fluoren-6-carbonsäureester erfolgt durch ESI-Massenspektrometrie.

**b) Optionale anschließende Darstellung der freien 5,6,6a,11b-Tetrahydro-7-oxa-5-aza-benzo[c]fluoren-6-carbonsäuren.**
Der zuvor beschriebene 5,6,6a,11b-Tetrahydro-7-oxa-5-aza-benzo[c]fluoren-6-carbonsäureester (1 Äquivalent) wird in 4 ml/mmol Ethanol gelöst und unter Rühren bei Raumtemperatur mit 1,2 Äquivalenten wässriger 6N Natronlauge versetzt. Der Verlauf der Ester-Verseifung wird durch Dünnschichtchromatographie verfolgt (Laufmittelsystem Diethylether /Hexan, 1:1) und ist nach 30 Minuten beendet (DC-Kontrolle). Das Reaktionsgemisch wird am Rotationsverdampfer eingeengt, in ca. 10 ml Wasser aufgenommen und mit 32 %-iger HCl auf pH 1 eingestellt. Die wäßrige Lösung wird fünf Mal mit Diethylether extrahiert und nach Trocknen über Magnesiumsulfat eingeengt.

**Automatisiertes Verfahren**

Ein Rundbodenröhrchen aus Glas (Durchmesser 16 mm, Länge 125 mm) mit Gewinde wurde mit einem Rührer versehen und mit einem Schraubdeckel mit Septum verschlossen. Das Röhrchen wurde in den auf 20 °C temperierten Rührblock gestellt. Anschließend wurden nacheinander die folgenden Reagenzien hinzupipettiert:

1 ml einer Lösung aus Trifluoressigsäure, 0,1 M, und Anilinkomponente, 0,1 M, in Acetonitril;
1 ml einer 0,11 M Lösung des Aldehyds in Acetonitril;
1 ml einer 0,3 M Lösung des Benzofurans in Acetonitril.

Dabei ist hier unter Aldehyd sowohl die Glyoxalsäure als auch der Glyoxalester, vorzugsweise der Alkylester, insbesondere der Ethyl- oder Methylester, zu verstehen. Das Reaktionsgemisch wurde bei 20 °C in einem der Rührblöcke 10 h lang gerührt. Danach wurde die Reaktionslösung abfiltriert. Das Röhrchen wurde dabei zweimal mit je 1,5 ml, einer 7,5% $NaHCO_3$-Lösung gespült.
Das Reaktionsgemisch wurde auf einem Vortexer mit 2 ml Ethylacetat versetzt und geschüttelt. Zur Ausbildung der Phasengrenze wurde in der Zentrifuge kurz zentrifugiert. Die Phasengrenze wurde optisch detektiert und die organische Phase abpipettiert. Im nächsten Schritt wurde die wäßrige Phase erneut mit 2 ml Ethylacetat versetzt, geschüttelt, zentrifugiert und die organische Phase abpipettiert. Die vereinigten organischen Phasen wurden über 2,4 g $MgSO_4$ (granuliert) getrocknet. Das Lösungsmittel wurde in einer Vakuumzentrifuge entfernt.
Die Charakterisierung der freien 5,6,6a,11b-Tetrahydro-7-oxa-5-aza-benzo[c]fluoren-6-carbonsäure oder des -esters erfolgt durch ESI-Massenspektrometrie.
Grundsätzlich kann sich bei Verbindungen mit R3 $\neq$ H sowohl nach automatisierten wie normalem Grundverfahren eine Verseifung nach dem Fachmann bekannten Methoden anschliessen, so beispielsweise mit KOH-Lösung bzw. NaOH-Lösung in Methanol oder Ethanol bei 0°C - 100°C, vorzugsweise 40°C - 60°C.

**Beispiel 1**

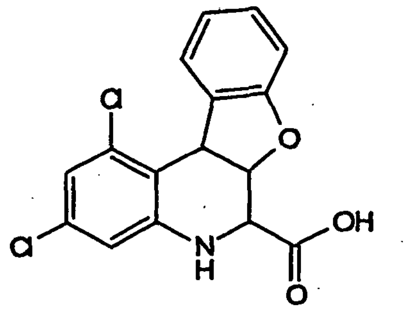

1,3-Dichlor-5,6,6a,11b-tetrahydro-7-oxa-5-aza-benzo[c]fluoren-6-carbonsäure (**1**)

Verbindung **1** wurde nach dem Grundverfahren nach Beispiel 0 aus 3,5-Dichloranilin, Glyoxalsäureethylester und dem unsubstituierten Benzofuran hergestellt, wobei der entstehende Ester abschließend verseift wurde.

**Beispiel 2**

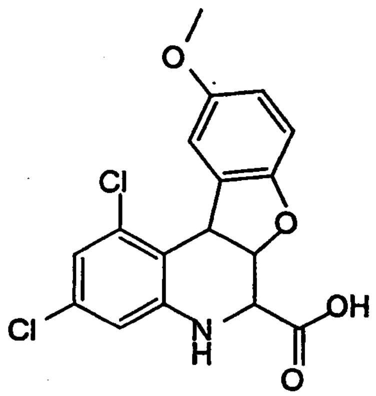

1,3-Dichlor-10-methoxy-5,6,6a,11b-tetrahydro-7-oxa-5-aza-benzo[c]fluoren-6-carbonsäure (**2**)

Verbindung **2** wurde nach dem Grundverfahren nach Beispiel 0 aus 3,5-Dichloranilin, Glyoxalsäureethylester und 5-Methoxybenzofuran hergestellt, wobei der entstehende Ester abschließend verseift wurde.

**Beispiel 3**

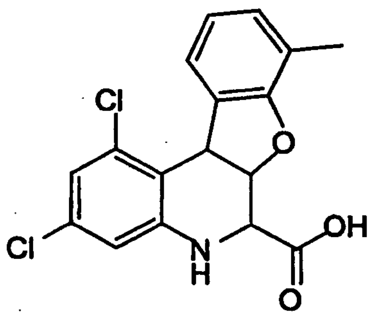

1,3-Dichlor-8-methyl-5,6,6a,11b-tetrahydro-7-oxa-5-aza-benzo[c]fluoren-6-carbonsäure (**3**)

Verbindung **3** wurde nach dem Grundverfahren nach Beispiel 0 aus 3,5-Dichloranilin, Glyoxalsäureethylester und 7-Methylbenzofuran hergestellt, wobei der entstehende Ester abschließend verseift wurde.

**Beispiel 4**

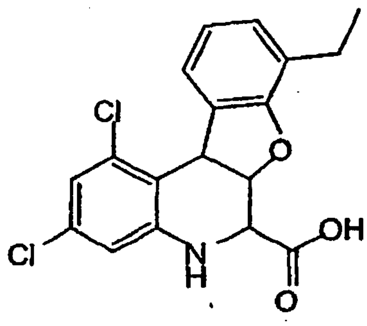

1,3-Dichlor-8-ethyl-5,6,6a,11b-tetrahydro-7-oxa-5-aza-benzo[c]fluoren-6-carbonsäure (**4**)

Verbindung **4** wurde nach dem Grundverfahren nach Beispiel 0 aus 3,5-Dichloranilin, Glyoxalsäureethylester und 7-Ethylbenzofuran hergestellt wobei der entstehende Ester abschließend verseift wurde.

**Beispiel 5**

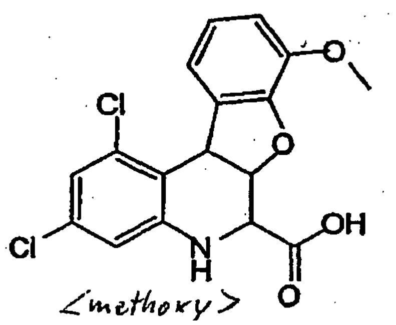


1,3-Dichlor-8-methoxy-5,6,6a,11b-tetrahydro-7-oxa-5-aza-benzo[c]fluoren-6-carbonsäure (**5**)

Verbindung **5** wurde nach dem Grundverfahren nach Beispiel 0 aus 3,5-Dichloranilin, Glyoxalsäureethylester und 7-Methoxybenzofuran hergestellt, wobei der entstehende Ester abschließend verseift wurde.

**Beispiel 6**

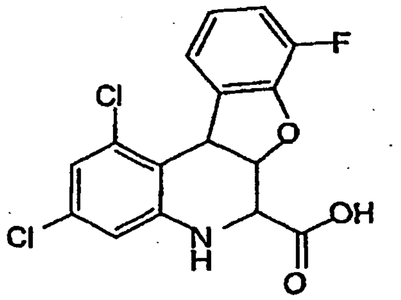

1,3-Dichlor-8-fluor-5,6,6a,11b-tetrahydro-7-oxa-5-aza-benzo[c]fluoren-6-carbonsäure (**6**)

Verbindung **6** wurde nach dem Grundverfahren nach Beispiel 0 aus 3,5-Dichloranilin, Glyoxalsäureethylester und 7-Fluorbenzofuran hergestellt, wobei der entstehende Ester abschließend verseift wurde.

**Beispiel 7**

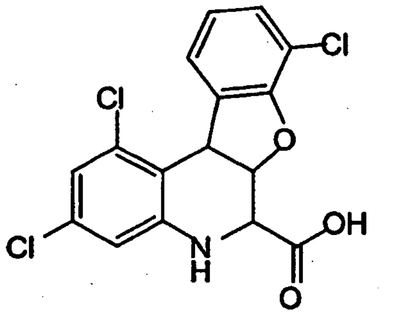

1,3,8-Trichlor-5,6,6a,11b-tetrahydro-7-oxa-5-aza-benzo[c]fluoren-6-carbonsäure (**7**)

Verbindung **7** wurde nach dem Grundverfahren nach Beispiel 0 aus 3,5-Dichloranilin, Glyoxalsäureethylester und 7-Chlorbenzofuran hergestellt, wobei der entstehende Ester abschließend verseift wurde.

**Beispiel 8**

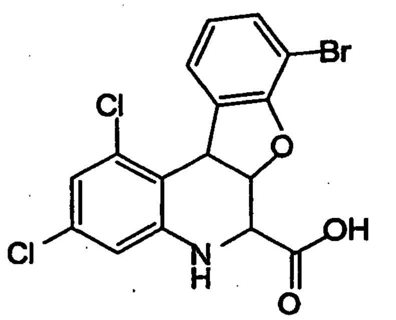

8-Brom-1,3-dichlor-5,6,6a,11b-tetrahydro-7-oxa-5-aza-benzo[c]fluoren-6-carbonsäure (**8**)

Verbindung **8** wurde nach dem Grundverfahren nach Beispiel 0 aus 3,5-Dichloranilin, Glyoxalsäureethylester und 7-Brombenzofuran hergestellt, wobei der entstehende Ester abschließend verseift wurde.

**Beispiel 9**

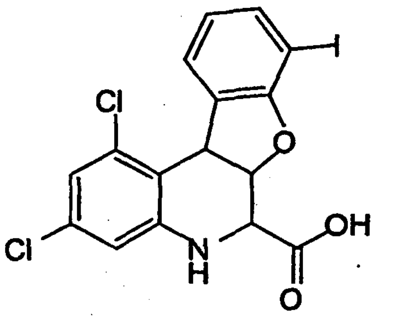

8-Iod-1,3-dichlor-5,6,6a,11b-tetrahydro-7-oxa-5-aza-benzo[c]fluoren-6-carbonsäure (**9**)

Verbindung **9** wurde nach dem Grundverfahren nach Beispiel 0 aus 3,5-Dichloranilin, Glyoxalsäureethylester und 7-Iodbenzofuran hergestellt, wobei der entstehende Ester abschließend verseift wurde.

**Beispiel 10**

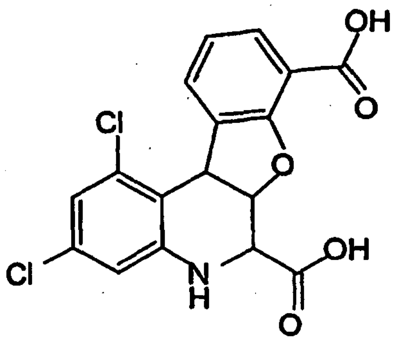

1,3-Dichlor-5,6,6a,11b-tetrahydro-7-oxa-5-aza-benzo[c]fluoren-6,8-dicarbonsäure (**10**)

Verbindung **10** wurde nach dem Grundverfahren nach Beispiel 0 aus 3,5-Dichloranilin, Glyoxalsäureethylester und Benzofuran-7-carbonsäure hergestellt, wobei der entstehende Ester abschließend verseift wurde.

**Beispiel 11**

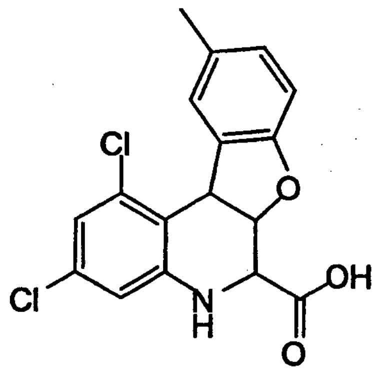

1,3-Dichlor-10-methyl-5,6,6a,11b-tetrahydro-7-oxa-5-aza-benzo[c]fluoren-6-carbonsäure (**11**)

Verbindung **11** nach dem Grundverfahren nach Beispiel 0 aus 3,5-Dichloranilin, Glyoxalsäureethylester und dem 5-Methylbenzofuran hergestellt, wobei der entstehende Ester abschließend verseift wurde.

**Beispiel 12**

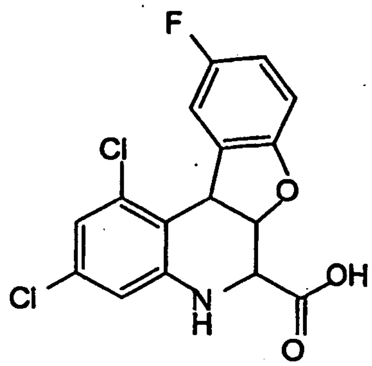

1,3-Dichlor-10-fluor-5,6,6a,11b-tetrahydro-7-oxa-5-aza-benzo[c]fluoren-6-carbonsäure (**12**)

Verbindung **12** wurde nach dem Grundverfahren nach Beispiel 0 aus 3,5-Dichloranilin, Glyoxalsäureethylester und 5-Fluorbenzofuran hergestellt, wobei der entstehende Ester abschließend verseift wurde.

**Beispiel 13**

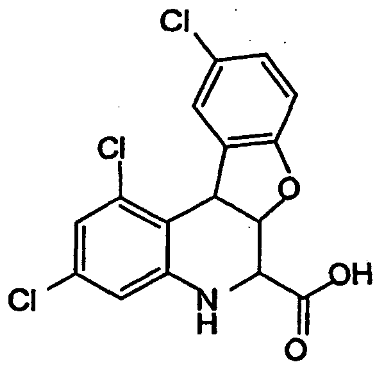

1,3,10-Trichlor-5,6,6a,11b-tetrahydro-7-oxa-5-aza-benzo[c]fluoren-6-carbonsäure (**13**)

Verbindung **13** wurde nach dem Grundverfahren nach Beispiel 0 aus 3,5-Dichloranilin, Glyoxalsäureethylester und 5-Chlorbenzofuran hergestellt, wobei der entstehende Ester abschließend verseift wurde.

**Beispiel 13**

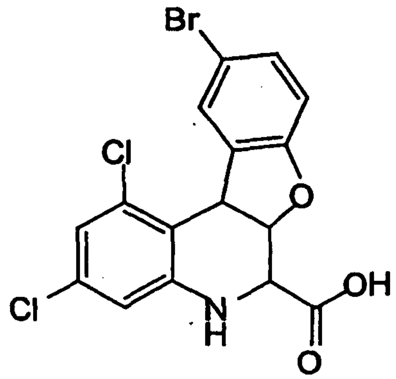

10-Brom-1,3-dichlor-5,6,6a,11b-tetrahydro-7-oxa-5-aza-benzo[c]fluoren-6-carbonsäure (**14**)

Verbindung 14 wurde nach dem Grundverfahren nach Beispiel 0 aus 3,5-Dichloranilin, Glyoxalsäureethylester und dem 5-Brombenzofuran hergestellt, wobei der entstehende Ester abschließend verseift wurde.

**Beispiel 14**

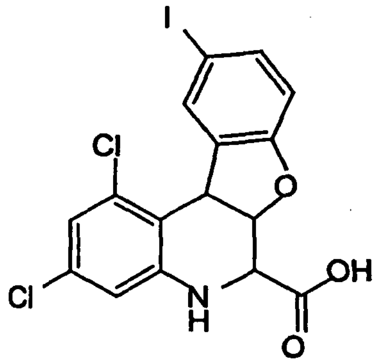

10-Iod-1,3-dichlor-5,6,6a,11b-tetrahydro-7-oxa-5-aza-benzo[c]fluoren-6-carbonsäure (**15**)

Verbindung **15** wurde nach dem Grundverfahren nach Beispiel 0 aus 3,5-Dichloranilin, dem Glyoxalsäureethylester und dem 5-Iodbenzofuran hergestellt, wobei der entstehende Ester abschließend verseift wurde.

**Beispiel 16**

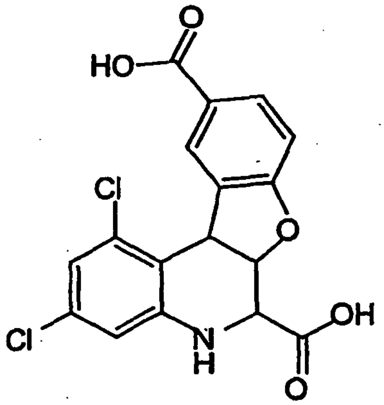

1,3-Dichlor-5,6,6a,11b-tetrahydro-7-oxa-5-aza-benzo[c]fluoren-6,10-dicarbonsäure (**16**)

Verbindung **16** wurde nach dem Grundverfahren nach Beispiel 0 aus 3,5-Dichloranilin, Glyoxalsäureethylester und Benzofuran-5-carbonsäure hergestellt, wobei der entstehende Ester abschließend verseift wurde.

**Beispiel 17**

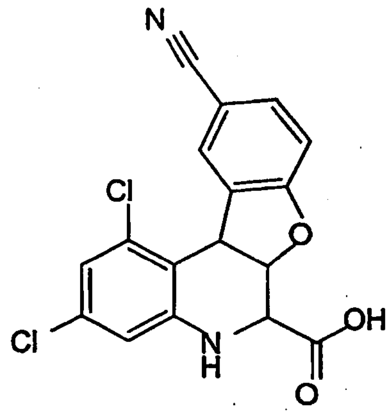

1,3-Dichlor-10-cyano-5,6,6a,11b-tetrahydro-7-oxa-5-aza-benzo[c]fluoren-6-carbonsäure (**17**)

Verbindung **17** wurde nach dem Grundverfahren nach Beispiel 0 aus 3,5-Dichloranilin, Glyoxalsäureethylester und 5-Cyanobenzofuran hergestellt, wobei der entstehende Ester abschließend verseift wurde.

**Beispiel 18: Allgemeine Darstellung der Salze mit Kationen am Beispiel der Verbindungen gemäß einem der Beispiele 1-17.**

Ein Äquivalent der Verbindung gemäß einem der Beispiele 1 bis 17, vorzugsweise eine Iminosäure, wird in wenig Wasser suspendiert und ein Äquivalent 1-normaler wäßriger Lauge, vorzugsweise NaOH oder KOH, zugegeben. Bei schlechter Löslichkeit wird soviel Methanol zugetropft, bis vollständige Lösung eintritt. Nach 30 Minuten Rühren bei Raumtemperatur wird im Rotationsverdampfer eingeengt, die verbliebene Lösung bei - 60°C in einem Gemisch aus Isopropanol/Trockeneis eingefroren und gefriergetrocknet. Die Salze, insbesondere der Iminosäuren, vorzugsweise die Natrium- oder Kaliumsalze, werden als meist farblose Feststoffe erhalten.

Alternativ ist es auch möglich, die Kalium- bzw. Natriumsalze mit Kalium-bzw. Natriumtrimethylsilanolat herzustellen (E.D. Laganis, B.L. Chenard; Tetrahedron Letters 25, 5831 - 5834 (1984)). Kalium- bzw. Natriumtrimethylsilanolat wird unter Stickstoff in einem organischen Lösungsmittel (Dichlormethan, Toluol, THF) gelöst und der Ester, bzw. die Säure, in einer Portion zugegeben. Das Reaktionsgemisch wird vier Stunden bei Raumtemperatur gerührt und abfiltriert. Der meist farblose Feststoff wird mit Diethylether gewaschen und im Vakuum getrocknet. Die Kalium- bzw. Natriumsalze werden als Feststoffe erhalten.

Die Verbindungen gemäß Beispiel 19 bis 35 sind nach diesen beispielhaften Vorschriften hergestellte Natriumsalze der Verbindungen 1 bis 17, die Verbindungen gemäß Beispielen 36 bis 52 die entsprechend hergestellten Kaliumsalze.

**Beispiel 19**

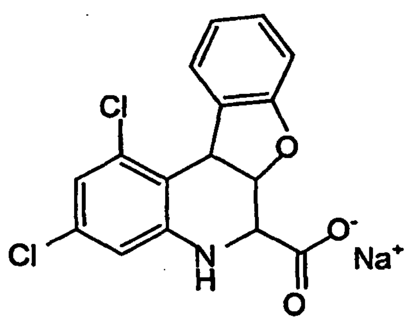

1,3-Dichlor-5,6,6a,11b-tetrahydro-7-oxa-5-aza-benzo[c]fluoren-6-carboxylat; Natriumsalz (**19**)

Verbindung **19** wurde nach dem Verfahren gemäß Beispiel **18** aus Verbindung **1** hergestellt.

**Beispiel 20**

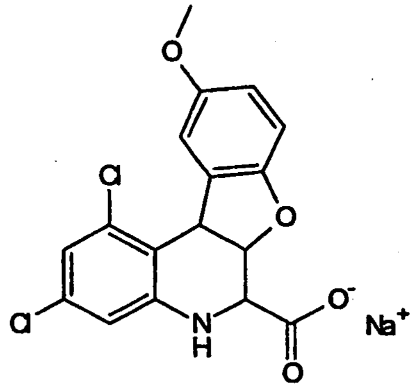

1,3-Dichlor-10-methoxy-5,6,6a,11b-tetrahydro-7-oxa-5-aza-benzo[c]fluoren-6-carboxylat; Natriumsalz (**20**)

Verbindung **20** wurde nach dem Verfahren gemäß Beispiel **18** aus Verbindung **2** hergestellt.

**Beispiel 21**

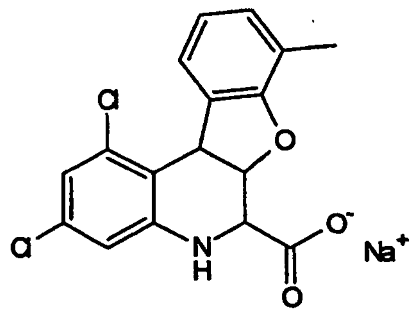

1,3-Dichlor-8-methyl-5,6,6a,11b-tetrahydro-7-oxa-5-aza-benzo[c]fluoren-6-carboxylat; Natriumsalz (**21**)

Verbindung **21** wurde nach dem Verfahren gemäß Beispiel **18** aus Verbindung **3** hergestellt.

**Beispiel 22**

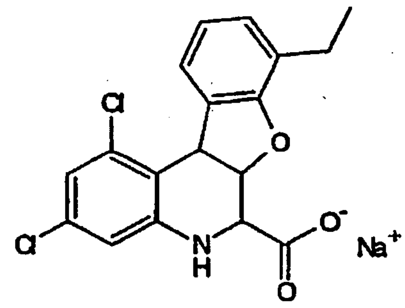

1,3-Dichlor-8-ethyl-5,6,6a,11b-tetrahydro-7-oxa-5-aza-benzo[c]fluoren-6-carboxylat; Natriumsalz (**22**)

Verbindung **22** wurde nach dem Verfahren gemäß Beispiel **18** aus Verbindung **4** hergestellt.

**Beispiel 23**

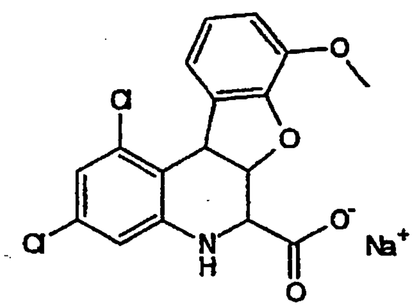

1,3-Dichlor-8-methoxy-5,6,6a,11b-tetrahydro-7-oxa-5-aza-benzo[c]fluoren-6-carboxylat; Natriumsalz (**23**)

Verbindung **23** wurde nach dem Verfahren gemäß Beispiel **18** aus Verbindung **5** hergestellt.

**Beispiel 24**

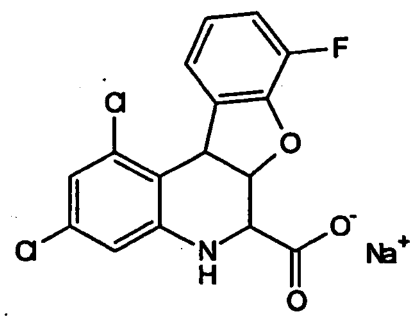

1,3-Dichlor-8-fluor-5,6,6a,11b-tetrahydro-7-oxa-5-aza-benzo[c]fluoren-6-carboxylat; Natriumsalz (**24**)

Verbindung **24** wurde nach dem Verfahren gemäß Beispiel **18** aus Verbindung **6** hergestellt.

**Beispiel 25**

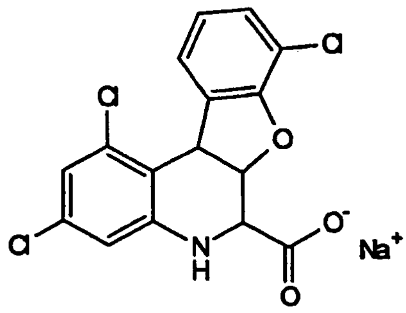

1,3,8-Trichlor-5,6,6a,11b-tetrahydro-7-oxa-5-aza-benzo[c]fluoren-6-carboxylat; Natriumsalz (**25**)

Verbindung **25** wurde nach dem Verfahren gemäß Beispiel **18** aus Verbindung **7** hergestellt.

**Beispiel 261**

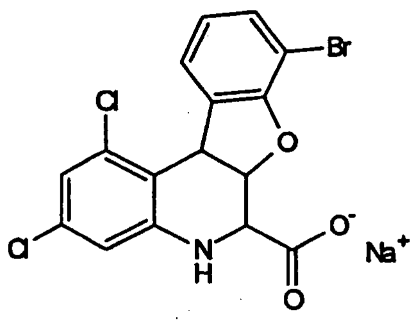

8-Brom-1,3-dichlor-5,6,6a,11b-tetrahydro-7-oxa-5-aza-benzo[c]fluoren-6-carboxylat; Natriumsalz (**26**)

Verbindung **26** wurde nach dem Verfahren gemäß Beispiel **18** aus Verbindung **8** hergestellt.

**Beispiel 27**

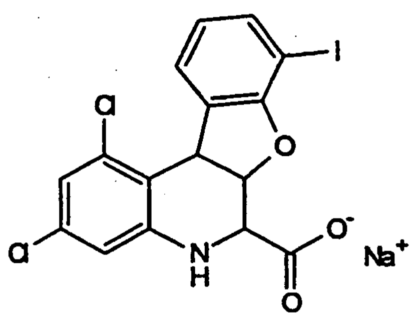

8-Iod-1,3-dichlor-5,6,6a,11b-tetrahydro-7-oxa-5-aza-benzo[c]fluoren-6-carboxylat; Natriumsalz (**27**)

Verbindung **27** wurde nach dem Verfahren gemäß Beispiel **18** aus Verbindung **9** hergestellt.

**Beispiel 28**

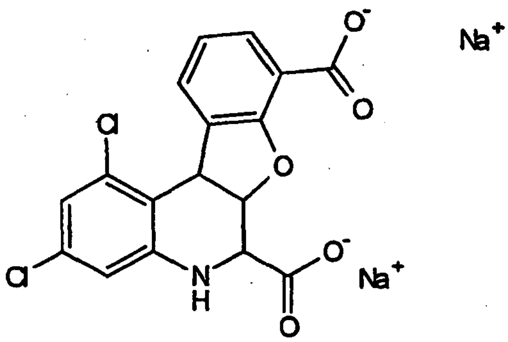

1,3-Dichlor-5,6,6a,11b-tetrahydro-7-oxa-5-aza-benzo[c]fluoren-6,8-dicarboxylat; Dinatriumsalz (**28**)

Verbindung **28** wurde nach dem Verfahren gemäß Beispiel **18** aus Verbindung **10** hergestellt.

**Beispiel 29**

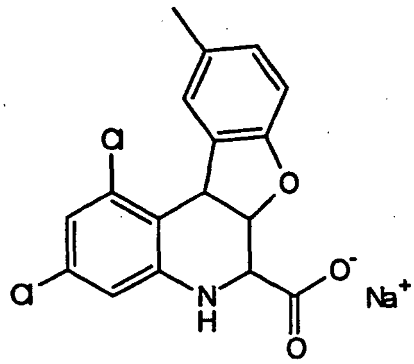

1,3-Dichlor-10-methyl-5,6,6a,11b-tetrahydro-7-oxa-5-aza-benzo[c]fluoren-6-carboxylat; Natriumsalz (**29**)

Verbindung **29** wurde nach dem Verfahren gemäß Beispiel **18** aus Verbindung **11** hergestellt.

**Beispiel 30**

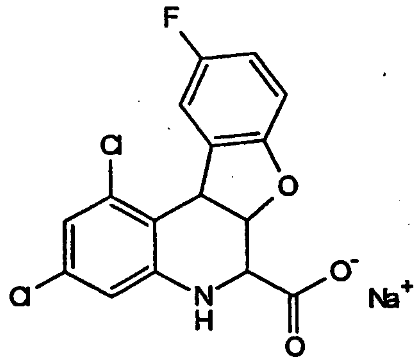

1,3-Dichlor-10-fluor 5,6,6a,11b-tetrahydro-7-oxa-5-aza-benzo[c]fluoren-6-carboxylat; Natriumsalz (**30**)

Verbindung **30** wurde nach dem Verfahren gemäß Beispiel **18** aus Verbindung **12** hergestellt.

**Beispiel 31**

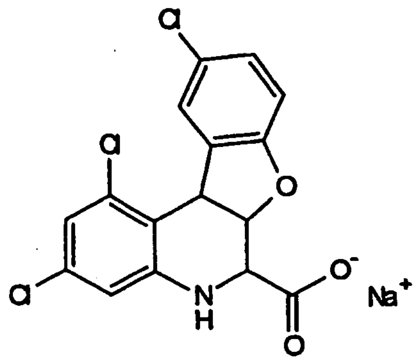

1,3,10-Trichlor-5,6,6a,11b-tetrahydro-7-oxa-5-aza-benzo[c]fluoren-6-carboxylat; Natriumsalz (**31**)

Verbindung **31** wurde nach dem Verfahren gemäß Beispiel **18** aus Verbindung **13** hergestellt.

**Beispiel 32**

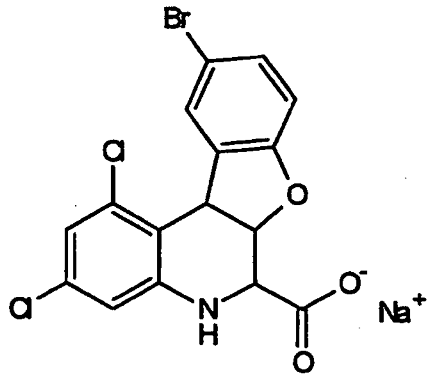

10-Brom-1,3-dichlor-5,6,6a,11b-tetrahydro-7-oxa-5-aza-benzo[c]fluoren-6-carboxylat; Natriumsalz (**32**)

Verbindung **32** wurde nach dem Verfahren gemäß Beispiel **18** aus Verbindung **14** hergestellt.

**Beispiel 33**

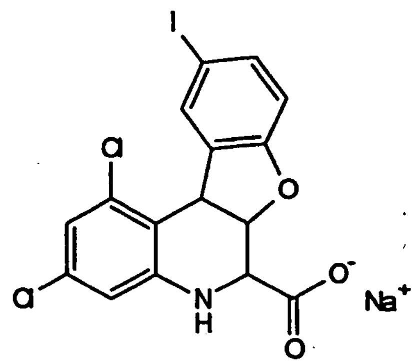

10-Iod-1,3-dichlor-5,6,6a,11b-tetrahydro-7-oxa-5-aza-benzo[c]fluoren-6-carboxylat; Natriumsalz (**33**)

Verbindung **33** wurde nach dem Verfahren gemäß Beispiel **18** aus Verbindung **15** hergestellt.

**Beispiel 34**

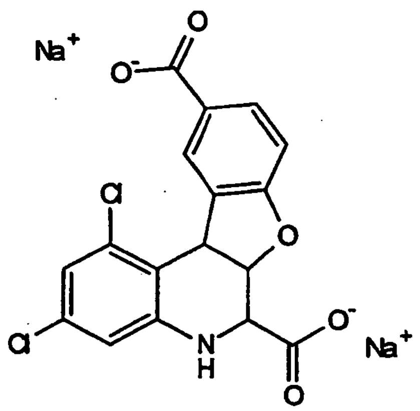

1,3-Dichior-5,6,6a,11b-tetrahydro-7-oxa-5-aza-benzo[c]fluoren-6,10-dicarboxylat; Dinatriumsalz (**34**)

Verbindung **34** wurde nach dem Verfahren gemäß Beispiel **18** aus Verbindung **16** hergestellt.

**Beispiel 35**

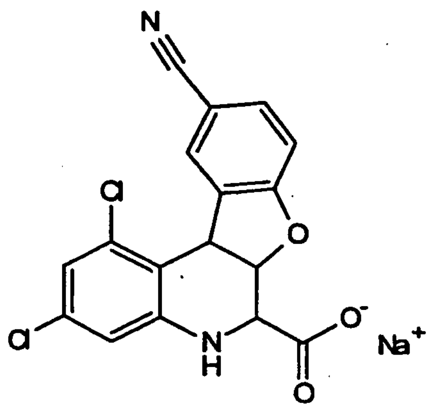

1,3-Dichlor-10-cyano-5,6,6a,11b-tetrahydro-7-oxa-5-aza-benzo[c]fluoren-6-carboxylat; Natriumsalz (**35**)

Verbindung **35** wurde nach dem Verfahren gemäß Beispiel **18** aus Verbindung **17** hergestellt.

**Beispiel 36**

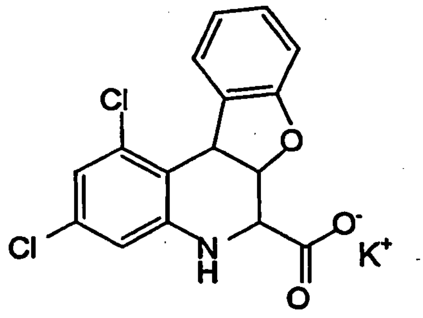

1,3-Dichlor-5,6,6a,11b-tetrahydro-7-oxa-5-aza-benzo[c]fluoren-6-carboxylat; Kaliumsalz (**36**)

Verbindung **36** wurde nach dem Verfahren gemäß Beispiel **18** aus Verbindung **1** hergestellt.

**Beispiel 37**

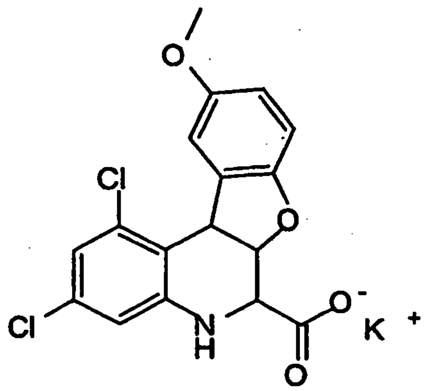

1,3-Dichlor-10-methoxy-5,6,6a,11b-tetrahydro-7-oxa-5-aza-benzo[c]fluoren-6-carboxylat; Kaliumsalz (**37**)

Verbindung **37** wurde nach dem Verfahren gemäß Beispiel **18** aus Verbindung **2** hergestellt.

**Beispiel 38**

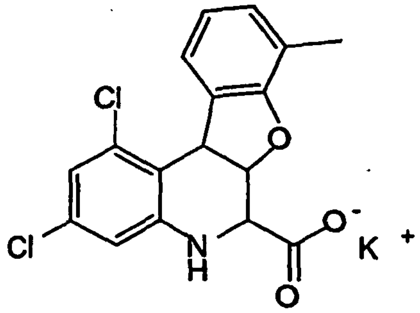

1,3-Dichlor-8-methyl-5,6,6a,11b-tetrahydro-7-oxa-5-aza-benzo[c]fluoren-6-carboxylat; Kaliumsalz (**38**)

Verbindung **38** wurde nach dem Verfahren gemäß Beispiel **18** aus Verbindung **3** hergestellt.

**Beispiel 39**

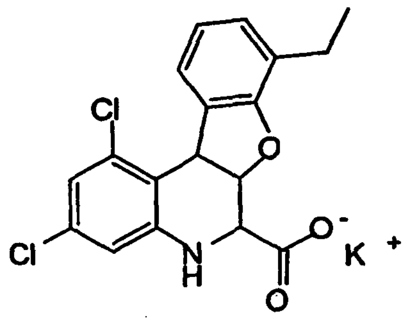

1,3-Dichfor-8-ethyl-5,6,6a,11b-tetrahydro-7-oxa-5-aza-benzo[c]fluoren-6-carboxylat; Kaliumsalz (**39**)

Verbindung **39** wurde nach dem Verfahren gemäß Beispiel **18** aus Verbindung **4** hergestellt.

**Beispiel 40**

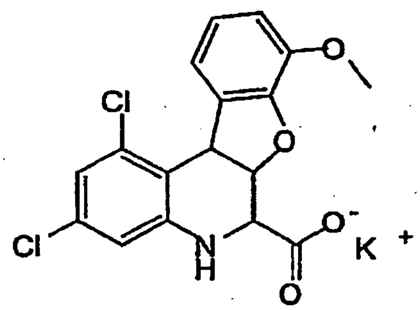

1,3-Dichlor-8-methoxy-5,6,6a,11b-tetrahydro-7-oxa-5-aza-benzo[c]fluoren-6-carboxylat; Kallumsalz (**40**)

Verbindung **40** wurde nach dem Verfahren gemäß Beispiel **18** aus Verbindung **5** hergestellt.

**Beispiel 41**

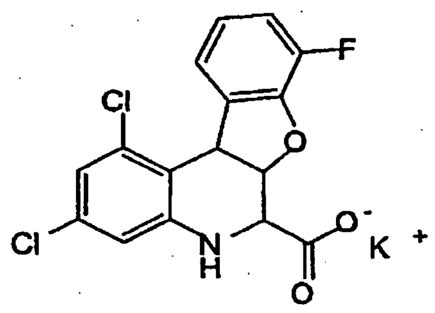

1,3-Dichlor-8-fluor-5,6,6a,11b-tetrahydro-7-oxa-5-aza-benzo[c]fluoren-6-carboxylat; Kaliumsalz (**41**)

Verbindung **41** wurde nach dem Verfahren gemäß Beispiel **18** aus Verbindung **6** hergestellt.

**Beispiel 42**

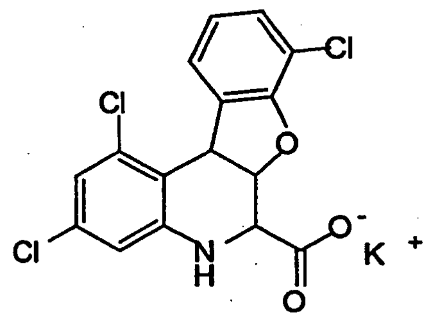

1,3,8-Trichlor-5,6,6a,11b-tetrahydro-7-oxa-5-aza-benzo[c]fluoren-6-carboxylat; Kaliumsalz (**42**)

Verbindung **42** wurde nach dem Verfahren gemäß Beispiel **18** aus Verbindung **7** hergestellt.

**Beispiel 43**

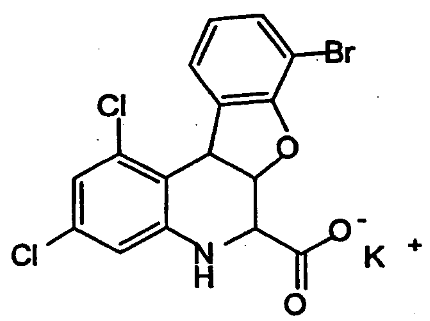

8-Brom-1,3-dichlor-5,6,6a,11b-tetrahydro-7-oxa-5-aza-benzo[c]fluoren-6-carboxylat; Kaliumsalz (**43**)

Verbindung **43** wurde nach dem Verfahren gemäß Beispiel **18** aus Verbindung **8** hergestellt.

**Beispiel 44**

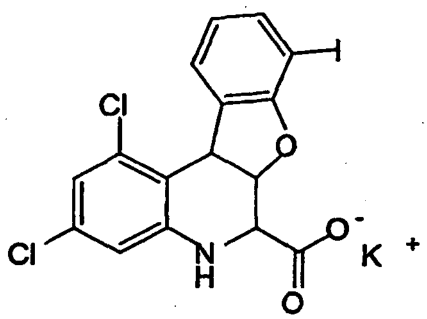

8-Iod-1,3-dichlor-5,6,6a,11b-tetrahydro-7-oxa-5-aza-benzo[c]fluoren-6-carboxylat; Kaliumsalz (**44**)

Verbindung **44** wurde nach dem Verfahren gemäß Beispiel **18** aus Verbindung **9** hergestellt.

**Beispiel 45**

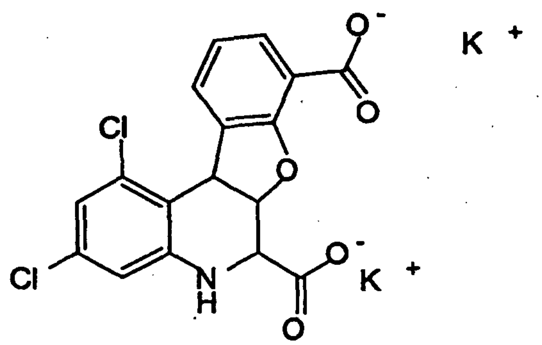

1,3-Dichlor-5,6,6a,11b-tetrahydro-7-oxa-5-aza-benzo[c]fluoren-6,8-dicarboxylat; Dikaliumsalz (**45**)

Verbindung **45** wurde nach dem Verfahren gemäß Beispiel **18** aus Verbindung **10** hergestellt.

**Beispiel 46**

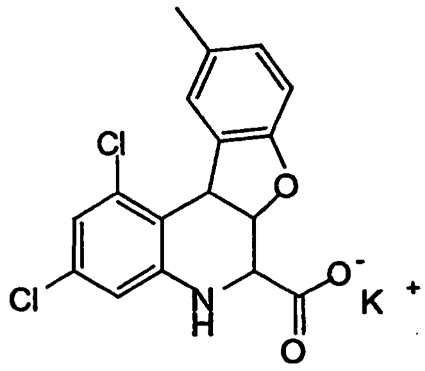

1,3-Dichlor-10-methyl-5,6,6a,11b-tetrahydro-7-oxa-5-aza-benzo[c]fluoren-6-carboxylat; Kaliumsalz (**46**)

Verbindung **46** wurde nach dem Verfahren gemäß Beispiel **18** aus Verbindung **11** hergestellt.

**Beispiel 47**

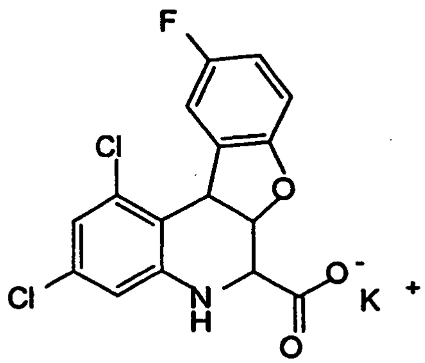

1,3-Dichlor-10-fluor-5,6,6a,11b-tetrahydro-7-oxa-5-aza-benzo[c]fluoren-6-carboxylat; Kaliumsalz (**47**)

Verbindung **47** wurde nach dem Verfahren gemäß Beispiel **18** aus Verbindung **12** hergestellt.

**Beispiel 48**

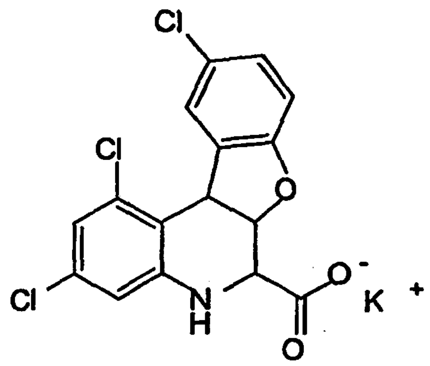

1,3,10-Trichlor-5,6,6a,11b-tetrahydro-7-oxa-5-aza-benzo[c]fluoren-6-carboxylat; Kaliumsalz (**48**)

Verbindung **31** wurde nach dem Verfahren gemäß Beispiel **18** aus Verbindung **13** hergestellt.

**Beispiel 49**

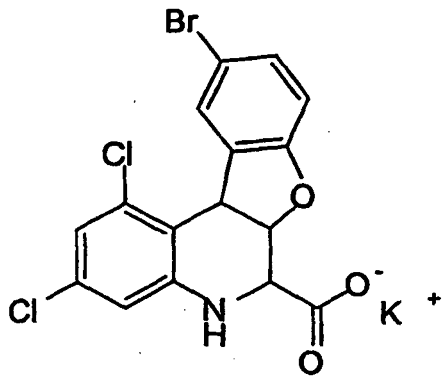

10-Brom-1,3-dichlor-5,6,6a,11b-tetrahydro-7-oxa-5-aza-benzo[c]fluoren-6-carboxylat; Kaliumsalz (**49**)

Verbindung **49** wurde nach dem Verfahren gemäß Beispiel **18** aus Verbindung **14** hergestellt.

**Beispiel 50**

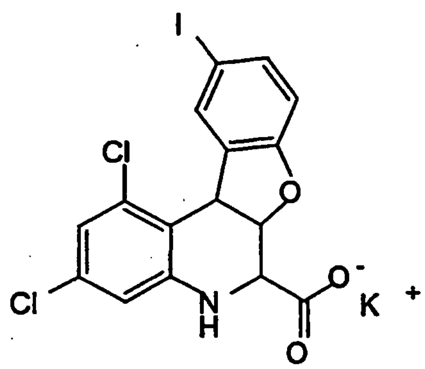

10-Iod-1,3-dichlor-5,6,6a,11b-tetrahydro-7-oxa-5-aza-benzo[c]fluoren-6-carboxylat; Kaliumsalz (**50**)

Verbindung **50** wurde nach dem Verfahren gemäß Beispiel **18** aus Verbindung **15** hergestellt.

**Beispiel 51**

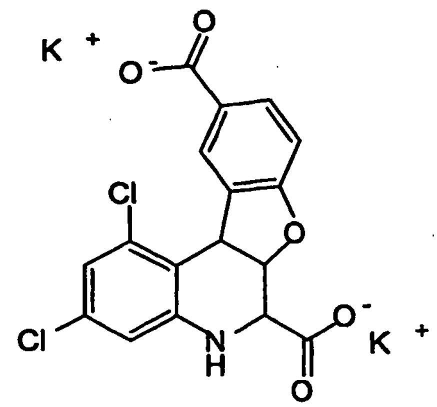

1,3-Dichlor-5,6,6a,11b-tetrahydro-7-oxa-5-aza-benzo[c]fluoren-6,10-dicarboxylat; Dikaliumsalz (**51**)

Verbindung **51** wurde nach dem Verfahren gemäß Beispiel **18** aus Verbindung **16** hergestellt.

**Beispiel 529**

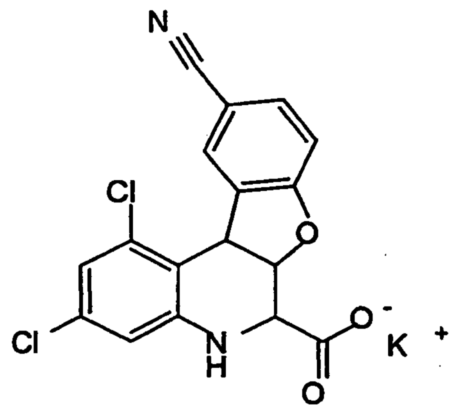

1,3-Dichlor-10-cyano-5,6,6a,11b-tetrahydro-7-oxa-5-aza-benzo[c]fluoren-6-carboxylat; Kaliumsalz (**52**)

Verbindung **52** wurde nach dem Verfahren gemäß Beispiel **18** aus Verbindung **17** hergestellt.

**Beispiel 53: Allgemeine Darstellung der Salze mit Anionen am Beispiel des Hydrochloridsalzes der Verbindungen gemäß einem der Beispiele 1-17.**

Ein Äquivalent der Verbindung gemäß einem der Beispiele 1 bis 17 wird in ca. 10 ml 2-Butanon je Gramm Substanz gelöst. Dann wird ein halbes Moläquivalent Wasser zugegeben, gefolgt von 1,1 Moläquivalenten Chlortrimethylsilan. Dann folgt Rühren über Nacht. Bildete sich auch bei Kühlung auf ca. 4 °C kein Hydrochlorid, wurde der

Fällungsansatz im doppelten Volumen Wasser aufgenommen, mit drei kleinen Portionen Ether gewaschen, die wäßrige Phase mit wenig ca. 30 %iger Natronlauge alkalisch gestellt und dreimal mit Ether extrahiert ("Säure-Base-Extraktion"). Diese letzten Extrakte wurden wiederum vereinigt und einer erneuten Hydrochloridfällung zugeführt.

Die Verbindungen gemäß Beispiel 54 bis 70 sind nach dieser beispielhaften Vorschrift hergestellte Hydrochloridsalze der Verbindungen 1 bis 17.

**Beispiel 54**

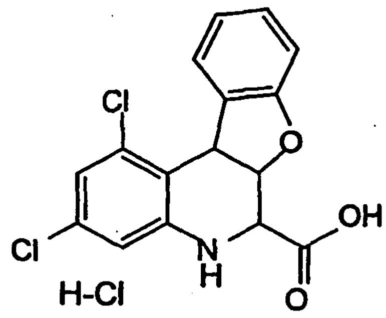

1,3-Dichlor-5,6,6a,11b-tetrahydro-7-oxa-5-aza-benzo[c]fluoren-6-carbonsäure; Hydrochloridsalz (**54**)

Verbindung **54** wurde nach dem Verfahren gemäß Beispiel **53** aus Verbindung **1** hergestellt.

**Beispiel 55**

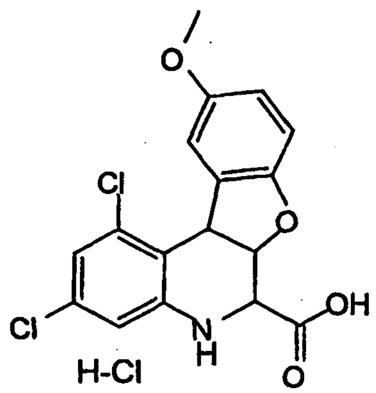

1,3-Dichlor-10-methoxy-5,6,6a,11b-tetrahydro-7-oxa-5-aza-benzo[c]fluoren-6-carbonsäure; Hydrochloridsalz (**55**)

Verbindung **55** wurde nach dem Verfahren gemäß Beispiel **53** aus Verbindung **2** hergestellt.

**Beispiel 56**

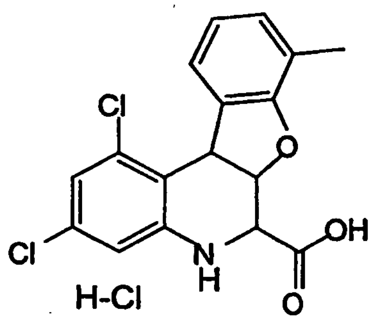

1,3-Dichlor-8-methyl-5,6,6a,11b-tetrahydro-7-oxa-5-aza-benzo[c]fluoren-6-carbonsäure; Hydrochloridsalz (**56**)

Verbindung **56** wurde nach dem Verfahren gemäß Beispiel **53** aus Verbindung **3** hergestellt.

**Beispiel 57**

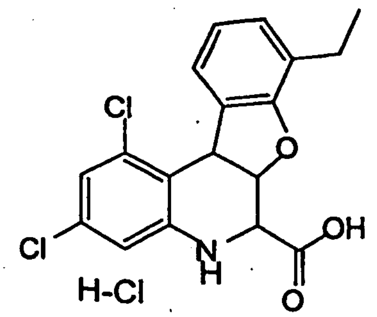

1,3-Dichlor-8-ethyl-5,6,6a,11b-tetrahydro-7-oxa-5-aza-benzo[c]fluoren-6-carbonsäure; Hydrochloridsalz (**57**)

Verbindung **57** wurde nach dem Verfahren gemäß Beispiel **53** aus Verbindung **4** hergestellt.

**Beispiel 58**

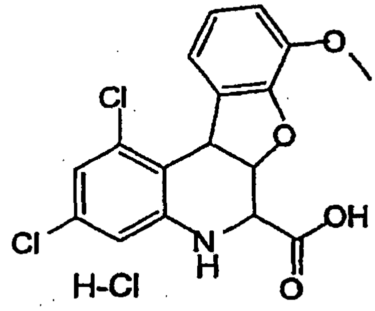

1,3-Dichlor-8-methoxy-5,6,6a,11b-tetrahydro-7-oxa-5-aza-benzo[c]fluoren-6-carbonsäure; Hydrochloridsalz (**58**)

Verbindung **58** wurde nach dem Verfahren gemäß Beispiel **53** aus Verbindung **5** hergestellt.

**Beispiel 59**

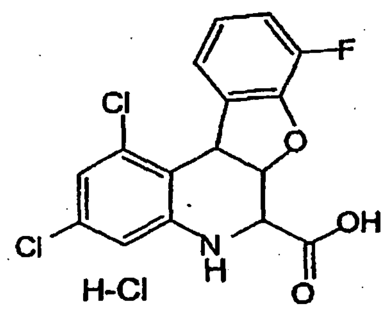

1,3-Dichlor-8-fluor-5,6,6a,11b-tetrahydro-7-oxa-5-aza-benzo[c]fluoren-6-carbonsäure; Hydrochloridsalz (**59**)

Verbindung **59** wurde nach dem Verfahren gemäß Beispiel **53** aus Verbindung **6** hergestellt.

**Beispiel 60**

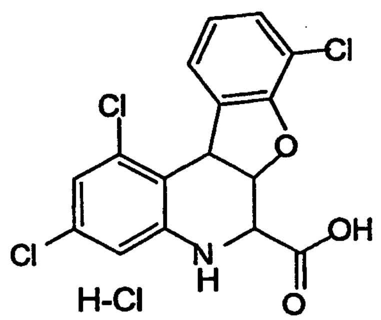

1,3,8-Trichlor-5,6,6a,11b-tetrahydro-7-oxa-5-aza-benzo[c]fluoren-6-carbonsäure; Hydrochloridsalz (**60**)

Verbindung **60** wurde nach dem Verfahren gemäß Beispiel **53** aus Verbindung **7** hergestellt.

**Beispiel 61**

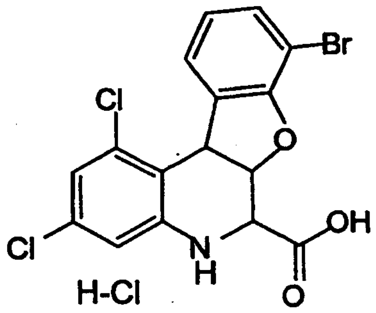

8-Brom-1,3-dichlor-5,6,6a,11b-tetrahydro-7-oxa-5-aza-benzo[c]fluoren-6-carbonsäure; Hydrochloridsalz (**61**)

Verbindung **61** wurde nach dem Verfahren gemäß Beispiel **53** aus Verbindung **8** hergestellt.

**Beispiel 62**

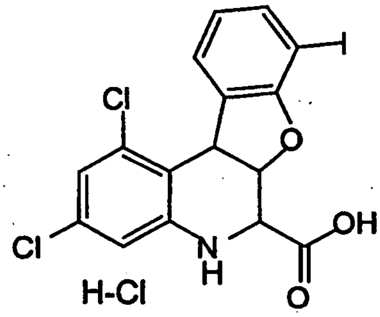

8-Iod-1,3-dichlor-5,6,6a,11b-tetrahydro-7-oxa-5-aza-benzo[c]fluoren-6-carbonsäure; Hydrochloridsalz (**62**)

Verbindung **62** wurde nach dem Verfahren gemäß Beispiel **53** aus Verbindung **9** hergestellt.

**Beispiel 63**

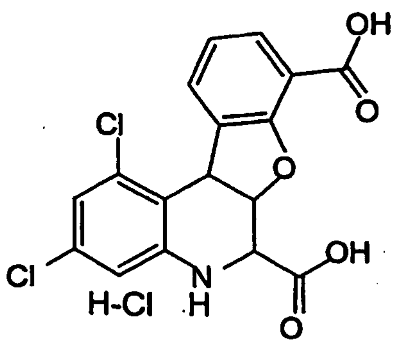

1,3-Dichlor-5,6,6a,11b-tetrahydro-7-oxa-5-aza-benzo[c]fluoren-6,8-dicarbonsäure; Hydrochloridsalz (**63**)

Verbindung **63** wurde nach dem Verfahren gemäß Beispiel **53** aus Verbindung **10** hergestellt.

**Beispiel 64**

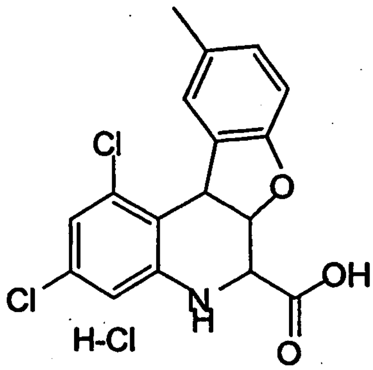

1,3-Dichlor-10-methyl-5,6,6a,11b-tetrahydro-7-oxa-5-aza-benzo[c]fluoren-6-carbonsäure; Hydrochloridsalz (**64**)

Verbindung **64** wurde nach dem Verfahren gemäß Beispiel **53** aus Verbindung **11** hergestellt.

**Beispiel 65**

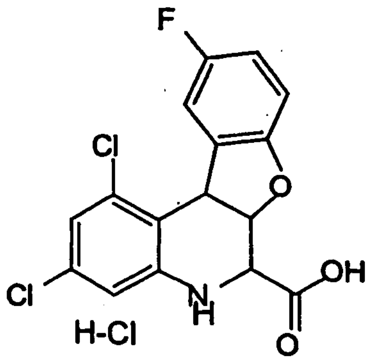

1,3-Dichlor-10-fluor-5,6,6a,11b-tetrahydro-7-oxa-5-aza-benzo[c]fluoren-6-carbonsäure; Hydrochloridsalz (**65**)

Verbindung **65** wurde nach dem Verfahren gemäß Beispiel **53** aus Verbindung **12** hergestellt.

**Beispiel 66**

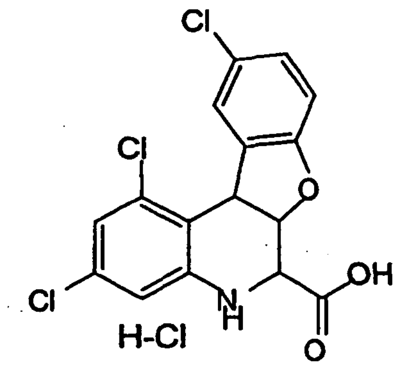

1,3,10-Trichlor-5,6,6a,11b-tetrahydro-7-oxa-5-aza-benzo[c]fluoren-6-carbonsäure; Hydrochloridsalz (**66**)

Verbindung **66** wurde nach dem Verfahren gemäß Beispiel **53** aus Verbindung **13** hergestellt.

**Beispiel 67**

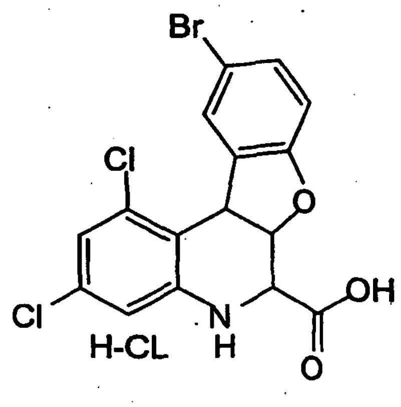

10-Brom-1,3-dichlor-5,6,6a,11b-tetrahydro-7-oxa-5-aza-benzo[c]fluoren-6-carbonsäure; Hydrochloridsalz (**67**)

Verbindung **67** wurde nach dem Verfahren gemäß Beispiel **53** aus Verbindung **14** hergestellt.

**Beispiel 68**

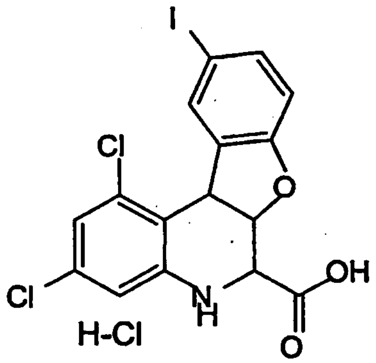

10-Iod-1,3-dichlor-5,6,6a,11b-tetrahydro-7-oxa-5-aza-benzo[c]fluoren-6-carbonsäure; Hydrochloridsalz (**68**)

Verbindung **68** wurde nach dem Verfahren gemäß Beispiel **53** aus Verbindung **15** hergestellt.

**Beispiel 69**

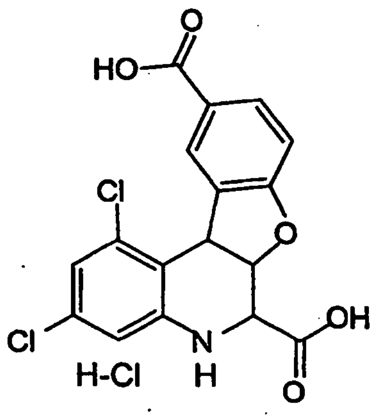

1,3-Dichlor-5,6,6a,11b-tetrahydro-7-oxa-5-aza-benzo[c]fluoren-6,10-dicarbonsäure; Hydrochloridsalz (**69**)

Verbindung **69** wurde nach dem Verfahren gemäß Beispiel **53** aus Verbindung **16** hergestellt.

**Beispiel 70**

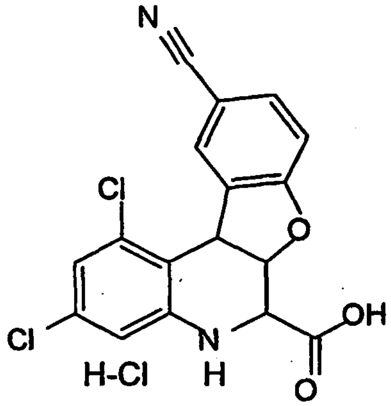

1,3-Dichlor-10-cyano-5,6,6a,11b-tetrahydro-7-oxa-5-aza-benzo[c]fluoren-6-carbonsäure; Hydrochloridsalz (**70**)

Verbindung **70** wurde nach dem Verfahren gemäß Beispiel **53** aus Verbindung **17** hergestellt.

**Beispiel 71**

Rezeptorbindung (Glycin-Bindungsstelle des NMDA-Rezeptorkanals)

Die Untersuchungen zur Bestimmung der Affinität der erfindungsgemäßen Verbindungen der Formel I zur Glycin-Bindungsstelle des NMDA-Rezeptorkanals wurde an Himmembran-Homogenaten (Homogenat von Cortex- und Hippocampus-Areal aus dem Him von männlichen Ratten, Stamm Wistar) durchgeführt [B.M. Baron , B.W. Siegel, B.L. Harrison, R.S. Gross, C. Hawes and P.Towers, Journal of Pharmacology and Experimental Therapeutics, Vol. 279, S. 62.1996].
Hierzu wurde Cortex und Hippocampus aus frisch entnommenen Rattengehimen freipräpariert und in 5 mmol/l TRIS-Acetatpuffer, 0,32 mol/l Sacharose pH 7,4 (10 ml/g Frischgewicht) mit einem Potter-Homogenisator (Fa. Braun/Melsungen 10 Kolbenhübe bei 500 Upm) unter Eiskühlung homogenisiert und anschließend für 10 Minuten bei 1.000 g und 4°C zentrifugiert. Der erste Überstand wurde gesammelt und das Sediment erneut mit 5 mmol/l TRIS-Acetatpuffer, 0,32 mol/l Sacharose pH 7,4 (5 ml/g ursprüngliches Frischgewicht) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wurde mit dem Überstand aus der ersten Zentrifugation vereinigt und bei 17.000 g für 20 Minuten bei 4°C zentrifugiert. Der Überstand nach dieser Zentrifugation wurde verworfen und das Membran-sediment mit 5 mmol/l TRIS-Acetatpuffer pH 8,0 (20 ml/g ursprüngliches Frischgewicht) aufgenommen und mit 10 Kolbenhüben bei 500 Upm homogenisiert. Anschließend wurde das Membranhomogenat für 1 Stunde bei 4°C inkubiert für 30 Minuten bei 50.000 g und 4°C zentrifugiert. Der Überstand wurde verworfen und das Zentrifugen-röhrchen mit dem Membransediment mit Parafilm verschlossen und für 24 Stunden bei -20°C eingefroren. Am folgenden Tag wurde das Membransediment aufgetaut und mit eiskaltem 5 mmol/l TRIS-Acetatpuffer, 0,1 % Saponin (w/v) pH 7,0 (10 ml/g ursprüngliches Frischgewicht) aufgenommen und mit 10 Kolbenhüben bei 500 Upm homogenisiert und anschließend für 20 Minuten bei 50.000 g und 4°C zentrifugiert. Der resultierende Überstand wurde verworfen und das Sediment in einem kleinen Volumen mit 5 mmo/l TRIS-Acetatpuffer pH 7,0 (ca. 2 ml/g ursprüngliches Frischgewicht) aufgenommen und erneut mit 10 Kolbenhüben bei 500 Upm homogenisiert. Nach Bestimmung des Proteingehaltes wurde das Membranhomogenat mit 5 mmol/l TRIS-Acetatpuffer pH 7,0 auf eine Proteinkonzentration von 10 mg Protein/ml eingestellt und in Aliquoten bis zur Testung eingefroren.
Für den Rezeptorbindungstest wurden Aliquote aufgetaut 1:10 mit 5 mmo/l TRIS-Acetatpuffer pH 7,0 verdünnt, mit 10 Kolbenhüben bei 500 Upm mit dem Potter-Homogenisator (10 Kolbenhübe bei 500 Upm) unter Eiskühlung homogenisiert und für 60 Minuten bei 55.000 g bei 4°C zentrifugiert. Der Überstand wurde dekantiert und das Membransediment mit eiskaltem 50 mmol/l TRIS-Acetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Von diesem Membranhomogenat wurden jeweils 100 µl je 1 ml-Ansatz im Rezeptorbindungstest eingesetzt (0,1 mg Protein/ml im Endansatz).
Im Bindungstest wurde als Puffer 50 mmol/l TRIS-Acetatpuffer pH 7,0 sowie als radioaktiver Ligand 1 nmol/l ($^3$H)-MDL 105.519 (B.M. Baron et al. 1996) eingesetzt. Der Anteil an unspezifischer Bindung wurde in Anwesenheit von 1 mmol/l Glycin bestimmt.

In weiteren Ansätzen wurden die erfindungsgemäßen Verbindungen in Konzentrationsreihen zugegeben und die Verdrängung des radioaktiven Liganden aus seiner spezifischen Bindung an die Glycin-Bindungsstelle des NMDA-Rezeptorkanals ermittelt. Die jeweiligen Dreifachansätze wurden über 120 Minuten bei 4°C inkubiert und anschließend zur Bestimmung des an das Membranhomogenat gebundenen radioaktiven Liganden mittels Filtration durch Glasfaser-Filtermatten (GF/B) geerntet. Die auf den Glasfaser-Filtern zurückgehaltene Radioaktivität wurde nach Zugabe von Szintillator im β-Counter gemessen.
Die Affinität der erfindungsgemäßen Verbindungen zur Glycin-Bindungsstelle des NMDA-Rezeptorkanals wurde als $IC_{50}$ (Konzentration mit 50 % Verdrängung des radioaktiven Liganden aus seiner spezifischen Bindung) nach dem Massenwirkungsgesetz mittels nichtlinearer Regression berechnet und ist in Tabelle 1 nach Umrechnung (nach der Cheng-Prussoff-Beziehung) als Ki-Wert (Mittelwert von 3 unabhängigen Versuchen) angegeben oder als prozentualer Anteil des zuvor gebundenen radioaktiven Liganden s.o., der bei einer Konzentration von 10 μmol/l der zu testenden erfindungsgemäßen Substanz aus seiner spezifischen Bindung verdrängt wird.

Tabelle **1**

| Beispiel | GlycinBindungsstelle des NMDA-Rezeptorkanals | |
|---|---|---|
| | Ki (μmol/l) | Verdrängung (%,10μmol/l) |
| **1** | **0,053** | **100** |

**Beispiel 72**

**Formalin-Test, Ratte**

Die Untersuchungen zur Bestimmung der antinociceptiven Wirkung der erfindungsgemäßen Verbindungen der Formel I wurden im Formalin-Test an männlichen Ratten (Sprague-Dawley, 150 -170 g) durchgeführt. Im Formalin-Test werden die erste (frühe) Phase (0-15 min nach Formalin-Injektion) und die zweite (späte) Phase (15 - 60 min nach Formalin-Injektion) unterschieden (D. Dubuisson, S.G. Dennis, Pain 4, 161 -174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T.J. Coderre, J. Katz, A.L. Vaccarino, R. Melzack, Pain, Vol. 52, S. 259, 1993). Die erfindungsgemäßen Verbindungen wurden in der zweiten Phase des Formalin-Tests untersucht, um Aussagen über Substanzwirkungen im chronisch/entzündlichen Schmerz zu erhalten. Durch eine einmalige subkutane Formalin-Injektion (50 μl, 5 %ig) in die dorsale Seite der rechten Hinterpfote wurde bei freibeweglichen Versuchstieren eine nociceptive Reaktion induziert, die sich in folgenden Verhaltensparametern darstellt: Heben und Halten der betroffenen Pfote (Score 1), Schütteln bzw. Zucken (Score 2), Lecken und Beißen (Score 3). Die aufgrund der Formalininjektion ausgelösten differierenden Verhaltensweisen wurden durch Beobachtung der Tiere in der späten Phase des Formalin-Tests kontinuierlich erfaßt und in einer Bewertung unterschiedlich gewichtet. Normalverhalten, bei dem das Tier alle vier Pfoten gleichmäßig belastet, wurde als Score 0 registriert. Abhängig von der Applikationsart der erfindungsgemäßen Verbindungen wurde der Applikationszeitpunkt vor der Formalin-Injektion gewählt (intraperitoneal: 15 min, intravenös: 5 min). Nach Injektion von Substanzen, die im Formalin-Test antinozizeptiv wirksam sind, sind die beschriebenen Verhaltensweisen (Score 1 - 3) der Tiere reduziert, evtl. sogar aufgehoben. Der Vergleich erfolgte mit Kontrolltieren, die Vehikel (Lösungsmittel) vor Formalinapplikation erhalten hatten. Das nozizeptive Verhalten wurde als sogenannte Schmerz-Rate (Pain-Rate, PR) berechnet. Die verschiedenen Verhaltensparameter erhielten eine unterschiedliche Gewichtung (Faktor 0, 1, 2, 3). Die Kalkulation erfolgte in Teilintervallen von 3 min nach folgender Formel:

$$PR = [(T_0 \times 0) + (T_1 \times 1) + (T_2 \times 2) + (T_3 \times 3)] / 180,$$

wobei $T_0$, $T_1$, $T_2$, und $T_3$ jeweils der Zeit in Sekunden entspricht, in der das Tier die Verhaltensweisen 0, 1, 2 oder 3 zeigte. Substanz- und Vehikelgruppen umfassen jeweils n = 10 Tiere. Basierend auf den PR-Berechnungen wurde die Substanzwirkung als Änderung gegen Kontrolle in Prozent ermittelt. Die $ED_{50}$-Berechnungen erfolgten mittels Regressionsanalyse.
Alle untersuchten erfindungsgemäßen Verbindungen zeigten eine mittelstarke bis starke Hemmung der durch Formalin induzierten Nociception.
Die Ergebnisse ausgewählter Untersuchungen im Formalin-Test Ratte sind in der nachfolgenden Tabelle zusammengefaßt.

Tabelle 2:

| Verbindung | Applikationsart | $ED_{50}$ |
|---|---|---|
| **1** | i.v. | 7,27 mg/kg |

**Beispiel 73: Parenterale Applikationsform.**

38,5 g der Verbindung **1** werden in 1 l Wasser für Injektionszwecke bei Raumtemperatur gelöst und anschließend durch Zugabe von wasserfreier Glukose für Injektionszwecke auf isotone Bedingungen eingestellt.

**Patentansprüche**

1. Substituierte 5,6,6a,11b-Tetrahydro-7-oxa-5-aza-benzo[c]fluoren-6-carbonsäurederivate der allgemeinen Formel **I**

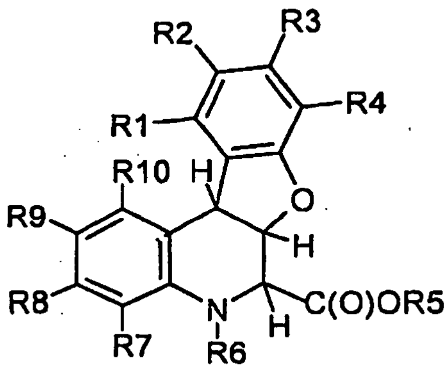

**I**

in dargestellter Form oder in Form ihrer Säuren oder ihrer Basen oder in Form ihrer Salze, oder in Form ihrer Solvate; in Form ihrer Racemate, ihrer reinen Stereoisomeren, oder in Form von Mischungen der Stereoisomeren, in einem beliebigen Mischungsverhältnis;
worin

**$R^1$**, **$R^2$**, **$R^3$** und **$R^4$** unabhängig voneinander ausgewählt sind aus H, F, Cl, Br, I; CN, $NH_2$, $CH_3$, $C_2H_5$, n-Propyl, i-Propyl, i-Butyl, sek-Butyl, n-Butyl, t-Butyl, $CF_3$, $CHF_2$, SH, OH, $OCH_3$, $OC_2H_5$, C(O)OH $C(O)OCH_3$ oder $C(O)OC_2H_5$
und
**$R^5$ = H,**
**$R^6$ = H,**
**$R^7$ = H,**
**$R^8$ = Cl,**
**$R^9$** = H und
**$R^{10}$** = Cl ist.

2. Substituierte 5,6,6a,11b-Tetrahydro-7-oxä-5-aza-benzo[c]fluoren-6-carbonsäurederivate gemäß Anspruch 1, **dadurch gekennzeichnet, daß** es sich um reine Enantiomere oder Diastereomere handelt, oder dass die Enantiomere oder Diastereomere in einem beliebigen Mischungsverhältnis vorliegen.

3. Substituierte 5,6,6a,11b-Tetrahydro-7-oxa-5-aza-benzo[c]fluoren-6-carbonsäurederivate gemäß Anspruch 1 oder 2, **dadurch gekennzeichnet, daß** es sich um folgende Verbindungen bzw. ihre Salze handelt:

- 1,3-Dichlor-5,6,6a,11b-tetrahydro-7-oxa-5-aza-benzo[c]fluoren-6-carbonsäure,
- 1,3-Dichlor-10-methoxy-5,6,6a,11b-tetrahydro-7-oxa-5-aza-benzo[c]fluoren-6-carbonsäure,
- 1,3-Dichlor-8-methyl-5,6,6a,11b-tetrahydro-7-oxa-5-aza-benzo[c]fluoren-6-carbonsäure,

- 1,3-Dichlor-8-ethyl-5,6,6a,11b-tetrahydro-7-oxa-5-aza-benzo[c]fluoren-6-carbonsäure,
- 1,3-Dichlor-8-fluor-5,6,6a,11b-tetrahydro-7-oxa-5-aza-benzo[c]fluoren-6-carbonsäure,
- 1,3,8-Trichlor-5,6,6a,11b-tetrahydro-7-oxa-5-aza-benzo[c]fluoren-6-carbonsäure,
- 8-Brom-1,3-dichlor-5,6,6a,11b-tetrahydro-7-oxa-5-aza-benzo[c]fluoren-6-carbonsäure,
- 8-Iod-1,3-dichlor-5,6,6a,11b-tetrahydro-7-oxa-5-aza-benzo[c]fluoren-6-carbonsäure,
- 1,3-Dichlor-5,6,6a,11b-tetrahydro-7-oxa-5-aza-benzo[c]fluoren-6,8-dicarbonsäure,
- 1,3-Dichlor-10-methyl-5,6,6a,11b-tetrahydro-7-oxa-5-aza-benzo[c]fluoren-6-carbonsäure,
- 1,3-Dichlor-10-fluor 5,6,6a,11b-tetrahydro-7-oxa-5-aza-benzo[c]fluoren-6-carbonsäure,
- 1,3,10-Trichlor-5,6,6a,11b-tetrahydro-7-oxa-5-aza-benzo[c]fluoren-6-carbonsäure,
- 10-Brom-1,3-dichlor-5,6,6a,11b-tetrahydro-7-oxa-5-aza-benzo[c]fluoren-6-carbonsäure,
- 10-Iod-1,3-dichlor-5,6,6a,11b-tetrahydro-7-oxa-5-aza-benzo[c]fluoren-6-carbonsäure,
- 1,3-Dichlor-5,6,6a,11b-tetrahydro-7-oxa-5-aza-benzo[c]fluoren-6,10-dicarbonsäure oder
- 1,3-Dichlor-10-cyano-5,6,6a, 11b-tetrahydro-7-oxa-5-aza-benzo[c]fluoren-6-carbonsäure.

**4.** Substituierte 5,6,6a,11b-Tetrahydro-7-oxa-5-aza-benzo[c]fluoren-6-carbonsäurederivate gemäß einem der Ansprüche 1 bis 3, **dadurch gekennzeichnet, daß** sie in Form ihrer Alkali-Salze, vorzugsweise der Kalium- oder Natrium--Salze, oder in Form der Salze anorganischer Säuren, vorzugsweise des Hydrochlorids, vorliegen.

**5.** Verfahren zur Herstellung von substituierten 5,6,6a,11b-Tetrahydro-7-oxa-5-aza-benzo[c]fluoren-6-carbonsäurederivaten gemäß Anspruch 1, **dadurch gekennzeichnet, daß** 3,5-Dichloranilin gemäß Formel II,

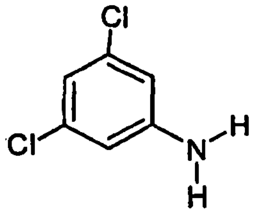

+

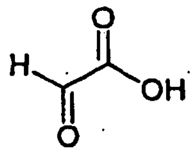

+

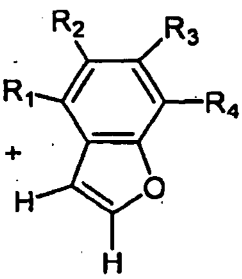

**II** **III** **IV**

mit Glyoxylsäure gemäß Formel **III** und einem Benzofuran gemäß **IV**, in denen $R^1$, $R^2$, $R^3$ und $R^4$ unabhängig voneinander jeweils eine der in Anspruch 1 angegebenen Bedeutungen haben, in Gegenwart von Trifluoressigsäure zwischen 0°C und 100°C umgesetzt werden.

**6.** Verfahren nach Anspruch 5, **dadurch gekennzeichnet, daß** die Reaktionsdauer 0.25 - 12 -Stunden, vorzugsweise maximal 2h, beträgt, die Reaktion bevorzugt bei Temperatur zwischen 20 und 40°C, vorzugsweise Raumtemperatur, erfolgt und/oder die Reaktion eine Eintopfreaktion ist.

**7.** Arzneimittel enthaltend als Wirkstoff mindestens ein substituiertes 5,6,6a,11b-Tetrahydro-7-oxa-5-aza-benzo[c]fluoren-6-carbonsäurederivat gemäß einem der Ansprüche 1 bis 3 sowie gegebenenfalls enthaltend geeignete Zusatz- und/oder Hilfsstoffe und/oder gegebenenfalls weitere Wirkstoffe.

**8.** Verwendung mindestens eines substituierten 5,6,6a,11b-Tetrahydro-7-oxa-5-aza-benzo[c]fluoren-6-carbonsäurederivats gemäß einem der Ansprüche 1 bis 3 zur Herstellung eines Arzneimittels zur Behandlung von Schmerz und/oder zur Behandlung von Migräne.

**9.** Verwendung mindestens eines substituierten 5,6,6a,11b-Tetrahydro-7-oxa-5-aza-benzo[c]fluoren-6-carbonsäurederivats gemäß Anspruch 8 zur Herstellung eines Arzneimittels zur Behandlung von neuropathischem und/oder chronischem Schmerz..

**10.** Verwendung mindestens eines substituierten 5,6,6a,11b-Tetrahydro-7-oxa-5-aza-benzo[c]fluoren-6-carbonsäurederivats gemäß einem der Ansprüche 1 bis 3 zur Herstellung eines Arzneimittels zur Behandlung von Harninkontinenz, Juckreiz, Tinnitus aurium und/oder Diarrhoe.

**11.** Verwendung mindestens eines substituierten 5,6,6a,11b-Tetrahydro-7-oxa-5-aza-benzo[c]fluoren-6-carbonsäurederivats gemäß einem der Ansprüche 1 bis 3 zur Herstellung eines Arzneimittels zur Behandlung/Prophylaxe von/bei Epilepsie, Morbus Parkinson, Morbus Huntington, Glaukom, Osteoporose, Ototoxizität, Entzugserscheinungen bei Alkohol- und/oder Drogenmißbrauch, Schlaganfall, cerebralen Ischämien, cerebralen Infarkten, Hirnödem, Hypoxie, Anoxie und/oder zur Anxiolyse und/oder Anästhesie.

## Claims

**1.** Substituted 5,6,6a,11b-tetrahydro-7-oxa-5-azabenzo[c]-fluorene-6-carboxylic acid derivatives of general formula I:

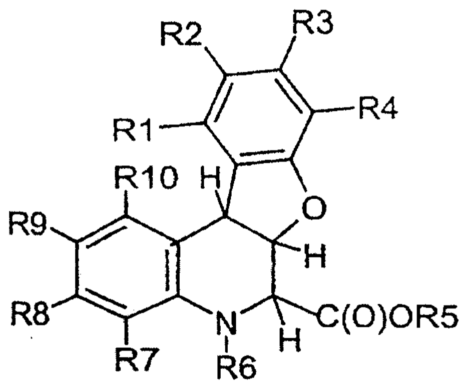

I

in the illustrated form, in the form of their acids or bases, in the form of their salts, in the form of their solvates, in the form of their racemates or their pure stereoisomers, or in the form of mixtures of the stereoisomers, in any desired mixing ratio,
in which formula

$R^1$, $R^2$, $R^3$ and $R^4$ independently of one another are selected from H, F, Cl, Br, I, CN, $NH_2$, $CH_3$, $C_2H_5$, n-propyl, i-propyl, i-butyl, sec-butyl, n-butyl, t-butyl, $CF_3$, $CHF_2$, SH, OH, $OCH_3$, $OC_2H_5$, C(O)OH, C(O)$OCH_3$ and C(O)$OC_2H_5$,
$R^5$ = H,
$R^6$ = H,
$R^7$= H,
$R^8$= Cl,
$R^9$= H and
$R^{10}$ = Cl.

**2.** Substituted 5,6,6a,11b-tetrahydro-7-oxa-5-azabenzo[c]-fluorene-6-carboxylic acid derivatives according to Claim 1, **characterized in that** they are pure enantiomers or diastereoisomers, or **in that** the enantiomers or diastereoisomers are in any desired mixing ratio.

**3.** Substituted 5,6,6a,11b-tetrahydro-7-oxa-5-azabenzo[c]-fluorene-6-carboxylic acid derivatives according to Claim 1 or 2, **characterized in that** they are the following compounds or their salts:

- 1,3-dichloro-5,6,6a,11b-tetrahydro-7-oxa-5-azabenzo-[c]fluorene-6-carboxylic acid,
- 1,3-dichloro-10-methoxy-5,6,6a,11b-tetrahydro-7-oxa-5-azabenzo[c]fluorene-6-carboxylic acid,
- 1,3-dichloro-8-methyl-5,6,6a,11b-tetrahydro-7-oxa-5-azabenzo[c]fluorene-6-carboxylic acid,
- 1,3-dichloro-8-ethyl-5,6,6a,11b-tetrahydro-7-oxa-5-azabenzo[c]fluorene-6-carboxylic acid,
- 1,3-dichloro-8-fluoro-5,6,6a,11b-tetrahydro-7-oxa-5-azabenzo[c]fluorene-6-carboxylic acid,
- 1,3,8-trichloro-5,6,6a,11b-tetrahydro-7-oxa-5-aza-benzo [c] fluorene-6-carboxylic acid,
- 8-bromo-1,3-dichloro-5,6,6a,11b-tetrahydro-7-oxa-5-azabenzo[c]fluorene-6-carboxylic acid,
- 8-iodo-1,3-dichloro-5,6,6a,11b-tetrahydro-7-oxa-5-azabenzo[c]fluorene-6-carboxylic acid,
- 1,3-dichloro-5,6,6a,11b-tetrahydro-7-oxa-5-azabenzo-[c]fluorene-6,8-dicarboxylic acid,

- 1,3-dichloro-10-methyl-5,6,6a,11b-tetrahydro-7-oxa-5-azabenzo[c]fluorene-6-carboxylic acid,
- 1,3-dichloro-10-fluoro-5,6,6a,11b-tetrahydro-7-oxa-5-azabenzo[c]fluorene-6-carboxylic acid,
- 1,3,10-trichloro-5,6,6a,11b-tetrahydro-7-oxa-5-aza-benzo[c]fluorene-6-carboxylic acid,
- 10-bromo-1,3-dichloro-5,6,6a,11b-tetrahydro-7-oxa-5-azabenzo[c]fluorene-6-carboxylic acid,
- 10-iodo-1,3-dichloro-5,6,6a,11b-tetrahydro-7-oxa-5-azabenzo[c]fluorene-6-carboxylic acid,
- 1,3-dichloro-5,6,6a,11b-tetrahydro-7-oxa-5-azabenzo-[c]fluorene-6,10-dicarboxylic acid or
- 1,3-dichloro-10-cyano-5,6,6a,11b-tetrahydro-7-oxa-5-azabenzo[c]fluorene-6-carboxylic acid.

**4.** Substituted 5,6,6a,11b-tetrahydro-7-oxa-5-azabenzo[c]-fluorene-6-carboxylic acid derivatives according to one of Claims 1 to 3, **characterized in that** they are in the form of their alkali metal salts, preferably the potassium of sodium salts, or in the form of the salts of inorganic acids, preferably the hydrochloride.

**5.** Process for the preparation of substituted 5,6,6a,11b-tetrahydro-7-oxa-5-azabenzo[c]fluorene-6-carboxylic acid derivatives according to Claim 1, **characterized in that** 3,5-dichloroaniline of formula II:

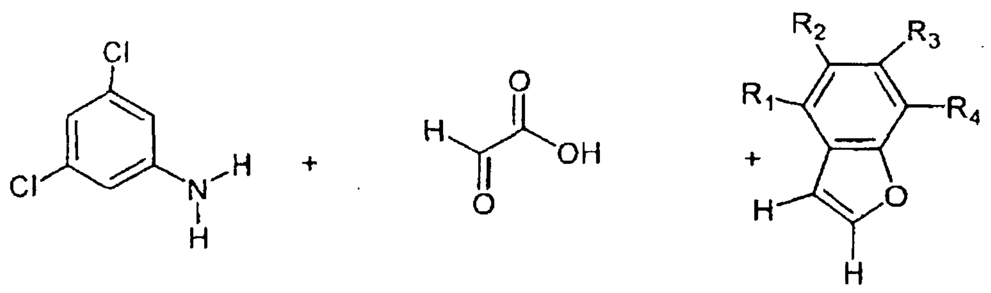

II III IV

is reacted with glyoxylic acid of formula III and a benzofuran IV, in which $R^1$, $R^2$, $R^3$ and $R^4$ independently of one another are each as defined in Claim 1, in the presence of trifluoroacetic acid at between 0°C and 100°C.

**6.** Process according to Claim 5, **characterized in that** the reaction time is 0.25 - 12 hours, preferably at most 2 h, and the reaction takes place preferably at a temperature between 20 and 40°C, particularly preferably at room temperature, and/or is a one-pot reaction.

**7.** Drug containing, as active substance, at least one substituted 5,6,6a,11b-tetrahydro-7-oxa-5-azabenzo[c]-fluorene-6-carboxylic acid derivative according to one of Claims 1 to 3, and optionally containing suitable additives and/or auxiliary substances and/or optionally other active substances.

**8.** Use of at least one substituted 5,6,6a,11b-tetrahydro-7-oxa-5-azabenzo[c]fluorene-6-carboxylic acid derivative according to one of Claims 1 to 3 for the preparation of a drug for the treatment of pain and/or for the treatment of migraine.

**9.** Use of at least one substituted 5,6,6a,11b-tetrahydro-7-oxa-5-azabenzo[c]fluorene-6-carboxylic acid derivative according to Claim 8 for the preparation of a drug for the treatment of neuropathic and/or chronic pain.

**10.** Use of at least one substituted 5,6,6a,11b-tetrahydro-7-oxa-5-azabenzo[c]fluorene-6-carboxylic acid derivative according to one of Claims 1 to 3 for the preparation of a drug for the treatment of urinary incontinence, itching, tinnitus aurium and/or diarrhoea.

**11.** Use of at least one substituted 5,6,6a,11b-tetrahydro-7-oxa-5-azabenzo[c]fluorene-6-carboxylic acid derivative according to one of Claims 1 to 3 for the preparation of a drug for the treatment/prophylaxis of epilepsy, Parkinson's disease, Huntington's disease, glaucoma, osteoporosis, ototoxicity, withdrawal symptoms associated with alcohol and/or drug abuse, stroke, cerebral ischaemia, cerebral infarction, cerebral oedema, hypoxia and anoxia, and/or for anxiolysis and/or anaesthesia.

## Revendications

**1.** Dérivés substitués d'acide 5,6,6a,11b-tétrahydro-7-oxa-5-aza-benzo[c]fluorène-6-carboxylique de formule générale I

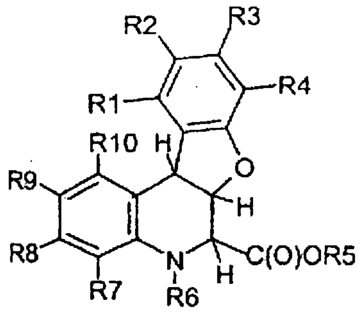

I

sous la forme représentée ou sous forme de leurs acides ou de leurs bases ou sous forme de leurs sels, ou sous forme de leurs produits de solvatation; sous forme de leurs racémates, de leurs stéréoisomères purs, ou sous forme de mélanges des stéréoisomères, dans un rapport de mélange quelconque ;
formule dans laquelle

$R^1$, $R^2$ ,$R^3$ et $R^4$ sont choisis, indépendamment les uns des autres, entre H, F, Cl, Br, I, CN, $NH_2$, $CH_3$, $C_2H_5$, n-propyle, isopropyle, isobutyle, sec.-butyle, n-butyle, tertiobutyle, $CF_3$, $CHF_2$, SH, OH, $OCH_3$, $OC_2H_5$, C(O)OH, $C(O)OCH_3$ ou $C(O)OC_2H_5$

et

$R^5$= H,
$R^6$ = H,
$R^7$= H,
$R^8$ = Cl
$R^9$= H et
$R^{10}$= Cl.

**2.** Dérivés substitués d'acide 5,6,6a,11b-tétrahydro-7-oxa-5-aza-benzo[c]fluorène-6-carboxylique suivant la revendication 1, **caractérisés en ce qu'**il s'agit d'énantiomères purs ou de diastéréoisomères purs, ou **en ce que** les énantiomères ou les diastéréoisomères sont présents dans un rapport de mélange quelconque.

**3.** Dérivés substitués d'acide 5,6,6a,11b-tétrahydro-7-oxa-5-aza-benzo[c]fluorène-6-carboxylique suivant la revendication 1 ou 2, **caractérisés en ce qu'**il s'agit des composés suivants ou de leurs sels :

- acide 1,3-dichloro-5,6,6a,11b-tétrahydro-7-oxa-5-aza-benzo[c]fluorène-6-carboxylique,
- acide 1,3-dichloro-10-méthoxy-5,6,6a,11b-tétrahydro-7-oxa-5-aza-benzo[c]fluorène-6-carboxylique,
- acide 1,3-dichloro-8-méthyl-5,6,6a,11b-tétrahydro-7-oxa-5-aza-benzo[c]fluorène-6-carboxylique,
- acide 1,3-dichloro-8-éthyl-5,6,6a,11b-tétrahydro-7-oxa-5-aza-benzo[c]fluorène-6-carboxylique,
- acide 1,3-dichloro-8-fluoro-5,6,6a,11b-tétrahydro-7-oxa-5-aza-benzo[c]fluorène-6-carboxylique,
- acide 1,3,8-trichloro-5,6,6a,11b-tétrahydro-7-oxa-5-aza-benzo[c]fluorène-6-carboxylique,
acide 8-bromo-1,3-dichloro-5,6,6a,11b-tétrahydro-7-oxa-5-aza-benzo[c]fluorène-6-carboxylique,
- acide 8-iodo-1,3-dichloro-5,6,6a,11b-tétrahydro-7-oxa-5-aza-benzo[c]fluorène-6-carboxylique,
- acide 1,3-dichloro-5,6,6a,11b-tétrahydro-7-oxa-5-aza-benzo[c]fluorène-6,8-dicarboxylique,
- acide 1,3-dichloro-10-méthyl-5,6,6a,11b-tétrahydro-7-oxa-5-aza-benzo[c]fluorène-6-carboxylique,
- acide 1,3-dichloro-10-fluoro-5,6,6a,11b-tétrahydro-7-oxa-5-aza-benzo[c]fluorène-6-carboxylique,
- acide 1,3,10-trichloro-5,6,6a,11b-tétrahydro-7-oxa-5-aza-benzo[c]fluorène-6-carboxylique,
- acide 10-bromo-1,3-dichloro-5,6,6a,11b-tétrahydro-7-oxa-5-aza-benzo[c]fluorène-6-carboxylique,
- acide 10-iodo-1,3-dichloro-5,6,6a,11b-tétrahydro-7-oxa-5-aza-benzo[c]fluorène-6-carboxylique,
- acide 1,3-dichloro-5,6,6a,llb-tétrahydro-7-oxa-5-aza-benzo[c]fluorène-6,10-dicarboxylique, ou

- acide 1,3-dichloro-10-cyano-5,6,6a,11b-tétrahydro-7-oxa-5-aza-benzo[c]fluorène-6-carboxylique.

4. Dérivés substitués d'acide 5,6,6a,11b-tétrahydro-7-oxa-5-aza-benzo[c]fluorène-6-carboxylique suivant l'une des revendications 1 à 3, **caractérisés en ce qu'**ils se présentent sous forme de leurs sels de métaux alcalins, avantageusement des sels de potassium ou de sodium, ou sous forme des sels d'acides inorganiques, avantageusement sous forme du chlorhydrate.

5. Procédé de production de dérivés substitués d'acide 5, 6, 6a, 11b-tétrahydro-7-oxa-5-aza-benzo [c] fluorène-6-carboxylique suivant la revendication 1, **caractérisé en ce qu'**on fait réagir la 3,5-dichloraniline de formule II

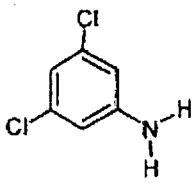
+
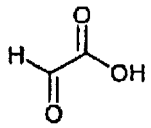
+
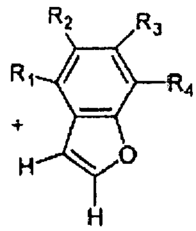

II III IV

avec l'acide glyoxylique de formule III et un benzofuranne de formule IV dans laquelle $R^1$, $R^2$, $R^3$ et $R^4$ ont chacun, indépendamment les uns des autres, l'une des définitions indiquées dans la revendication 1, en présence d'acide trifluoracétique, entre 0°C et 100°C.

6. Procédé suivant la revendication 5, **caractérisé en ce que** la durée de la réaction est de 0,25 à 12 heures, avantageusement de 2 heures au maximum, la réaction étant avantageusement conduite à une température comprise entre 20 et 40°C, avantageusement à la température ambiante, et/ou la réaction est une réaction en récipient unique.

7. médicament contenant, comme substance active, au moins un dérivé substitué d'acide 5,6,6a,11b-tétrahydro-7-oxa-5-aza-benzo[c]fluorène-6-carboxylique selon l'une des revendications 1 à 3 et contenant aussi, le cas échéant, des additifs et/ou des substances auxiliaires convenables et/ou le cas échéant d'autres substances actives.

8. Utilisation d'au moins un dérivé substitué d'acide 5,6,6a,11b-tétrahydro-7-oxa-5-aza-benzo[c]fluorène-6-carboxylique selon l'une des revendications 1 à 3 pour la préparation d'un médicament destiné au traitement de la douleur et/ou au traitement de la migraine.

9. Utilisation d'au moins un dérivé substitué d'acide 5,6,6a,11b-tétrahydro-7-oxa-5-aza-benzo[c]fluorène-6-carboxylique suivant la revendication 8 pour la préparation d'un médicament destiné au traitement d'une douleur neuropathique et/ou chronique.

10. Utilisation d'au moins un dérivé substitué d'acide 5,6,6a,11b-tétrahydro-7-oxa-5-aza-benzo[c] fluorène-6-carboxylique suivant l'une des revendications 1 à 3 pour la préparation d'un médicament destiné au traitement de l'incontinence urinaire, du prurit, des acouphènes et/ou de la diarrhée.

11. Utilisation d'au moins un dérivé substitué d'acide 5, 6, 6a, 11b-tétrahydro-7-oxa-5-aza-benzo [c] fluorène-6-carboxylique suivant l'une des revendications 1 à 3 pour la préparation d'un médicament destiné au traitement curatif /préventif de l'épilepsie, la maladie de Parkinson, la maladie de Huntington, le glaucome, l'ostéoporose, l'ototoxicité, des phénomènes de privation en cas d'abus d'alcool et/ou de drogue, de l'apoplexie cérébrale, d'ischémies cérébrales, d'infarctus cérébraux, d'oedème cérébral, d'hypoxie, d'anoxie et/ou en vue de l'anxiolyse et/ou de l'anesthésie.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 386839 A
- WO 9712879 A1
- WO 9807704 A1
- WO 9842673 A1
- WO 9834111 A
- WO 9842673 A


### In der Beschreibung aufgeführte Nicht-Patentliteratur


- *J. Med. Chem.,* 1992, vol. 35, 1954-1968
- *J. Med. Chem.,* 1992, vol. 35, 1942-1953
- *Med. Chem. Res.,* 1991, vol. 1, 64-73
- *Bioorganic and Medicinal Chemistry Lettetters,* 1992, vol. 2, 371
- *Journal of Heterocyclic Chemistry,* 1988, vol. 25, 1831
- *Journal of the Chemical Society, Perkin Transactions,* 1989, vol. I, 2245
- *Journal of the Chemical Society, Chemical Communications,* 1999, 651
- *Journal of the American Chemical Society,* 1996, vol. 118, 8977
- **E.D. LAGANIS ; B.L. CHENARD.** *Tetrahedron Letters,* 1984, vol. 25, 5831-5834
- **B.M. BARON ; B.W. SIEGEL ; B.L. HARRISON ; R.S. GROSS ; C. HAWES ; P.TOWERS.** *Journal of Pharmacology and Experimental Therapeutics,* 1996, vol. 279, 62
- **D. DUBUISSON ; S.G. DENNIS.** *Pain,* 1977, vol. 4, 161-174
- **T.J. CODERRE ; J. KATZ ; A.L. VACCARINO ; R. MELZACK.** *Pain,* 1993, vol. 52, 259